# EUROPÄISCHE PATENTSCHRIFT

(11) **EP 3 423 895 B1**
(45) Veröffentlichungstag und Bekanntmachung des Hinweises auf die Patenterteilung: **28.04.2021**
(21) Anmeldenummer: 16707637.1
(22) Anmeldetag: 02.03.2016
(51) Int. Cl.: G02F 1/1335, H01L 51/52, B29D 11/00, B33Y 80/00, B33Y 10/00, G02F 1/1333

(54) **VERFAHREN ZUR HERSTELLUNG EINER ANZEIGEVORRICHTUNG UND ANZEIGEVORRICHTUNG**
METHOD FOR PRODUCING A DISPLAY DEVICE, AND DISPLAY DEVICE
PROCÉDÉ POUR LA FABRICATION D'UN DISPOSITIF D'AFFICHAGE ET DISPOSITIF D'AFFICHAGE

(43) Veröffentlichungstag der Anmeldung: 09.01.2019
(73) Patentinhaber: PA.Cotte Family Holding GmbH, 92224 Amberg (DE)
(72) Erfinder: COTTE, Pierre-Alain, 92224 Amberg (DE)
(74) Vertreter: reuteler & cie SA
(86) Internationale Anmeldenummer: PCT/EP2016/000365
(87) Internationale Veröffentlichungsnummer: WO 2017/148491

(56) Entgegenhaltungen:
- EP-A2- 0 992 833
- WO-A1-2005/059636
- US-A1- 2011 157 887
- U. Füssel ET AL: "Fertigungstechnik - Trennen (1)", , 1. Dezember 2014 (2014-12-01), Seiten 1-38, XP055351845, Gefunden im Internet: URL:https://tu-dresden.de/ing/maschinenwes en/if/fue/ressourcen/dateien/studium/lehru nterlagen/ft_e/zat_1_2014?lang=en [gefunden am 2017-03-06]
- HAGINOYA CHISEKI ET AL: "Nanostructure array fabrication with a size-controllable natural lithography", APPLIED PHYSICS LETTERS, A I P PUBLISHING LLC, US, Bd. 71, Nr. 20, 17. November 1997 (1997-11-17), Seiten 2934-2936, XP012019087, ISSN: 0003-6951, DOI: 10.1063/1.120220
- Erich Müller: "Focused-Ion-Beam (FIB) Mikroskopie und Nanostrukturierung", , 4. Juni 2012 (2012-06-04), Seiten 1-2, XP055322087, Gefunden im Internet: URL:https://www.lem.kit.edu/192.php [gefunden am 2016-11-23]
- COTEC GMBH: "Nanotechnologie ? f?r absolut hochwertige Oberfl?chen", EASY-TO-CLEAN DURALON ULTRATECH, COTEC GMBH TECHNICAL ALTERATIONS RESERVED, 18. Mai 2011 (2011-05-18), Seiten 1-2, XP007918663, [gefunden am 2011-05-18]

## Beschreibung

Die Erfindung betrifft ein Verfahren zur Herstellung einer dünnen und im Wesentlichen bruchfesten Anzeigevorrichtung mit einem Display, wobei an lichtemittierenden Leuchtflächen des Displays eine Oberschicht angeordnet wird, die eine zu einem Betrachter gewandte Oberfläche aufweist. Ferner betrifft die Erfindung eine Anzeigevorrichtung. Anzeigevorrichtungen mit einer zu einem Betrachter gewandten Oberfläche aus Glas sind im Stand der Technik z.B. als LCD-Anzeigevorrichtungen oder OLED-Anzeigevorrichtungen, insbesondere bei Tablets oder Mobiltelefonen, bekannt. Nachteilig an solchen Anzeigevorrichtungen sind der begrenzte Kontrastumfang, den diese Anzeigevorrichtungen aufweisen, sowie die notwendige Dicke des Schutzglases dieser Anzeigevorrichtungen.

Daher ist es Aufgabe der vorliegenden Erfindung, ein Verfahren zur Herstellung einer Anzeigevorrichtung mit einem Display mit mehreren Pixeln bzw. lichtemittierenden Leuchtflächen, z. B. LCD- oder OLED-Anzeigevorrichtungen, hinsichtlich des Kontrastumfangs und/oder hinsichtlich der Anzeigequalität und/oder hinsichtlich des visuellen Eindrucks einer solchen Anzeige zu verbessern und/oder die Anzeigevorrichtung energieeffizienter zu gestalten und/oder das Gewicht einer Anzeigevorrichtung zu optimieren und/oder die mechanische Stabilität zu verbessern.

Die Aufgabe wird durch den unabhängigen Anspruch 1 gelöst. Vorteilhafte Weiterbildungen sind in den Unteransprüchen definiert

Ein Verfahren zur Herstellung einer dünnen und im Wesentlichen bruchfesten Anzeigevorrichtung mit einem Display gemäß des Oberbegriffs des Anspruchs 1 ist aus WO2005/059636 A1 bekannt.

Insbesondere wird die Aufgabe gelöst durch ein Verfahren zur Herstellung einer dünnen und im Wesentlichen bruchfesten Anzeigevorrichtung mit einem Display.

Dabei umfasst das Verfahren vorzugsweise, dass an lichtemittierenden Leuchtflächen des Displays eine Oberschicht angeordnet wird, die eine zu einem Betrachter gewandte Oberfläche aufweist, wobei vorzugsweise die Oberschicht im Wesentlichen Metall und/oder ein undurchsichtiges und/oder ein nicht-lichtreflektierendes Material umfasst. Dabei kann die Oberschicht mittelbar oder unmittelbar an lichtemittierenden Leuchtflächen des Displays angeordnet werden. Dadurch unterscheidet sich nicht nur das Verfahren zur Herstellung eines Displays grundsätzlich von herkömmlichen Herstellungsverfahren, sondern auch die damit hergestellte Anzeigevorrichtung. Denn während herkömmliche Anzeigevorrichtung eine im Wesentlichen aus Glas bestehende Oberschicht aufweisen, die Licht von einem Display zum einem Betrachter passieren lässt, kann - vereinfacht ausgedrückt - diese transparente Glasschicht aus dem Stand der Technik durch eine im Wesentlichen undurchsichtige Oberschicht aus Metall und/oder durch ein undurchsichtiges und/oder durch ein nicht-lichtreflektierendes Material ersetzt werden. Der Vorteil beim Ersetzen des Glases durch Metall und/oder ein undurchsichtiges und/oder nicht-lichtreflektierendes Material liegt zunächst darin, dass beispielsweise Metall eine deutlich höhere mechanische Stabilität aufweist als Glas, insbesondere hinsichtlich der Bruchfestigkeit.

Unter einem "Display" wird in der vorliegenden Beschreibung vorzugsweise eine elektrisch angesteuerte Anzeige ohne bewegliche Teile zur optischen Signalisierung von veränderlichen Informationen wie Bildern oder Zeichen verstanden. Günstigerweise werden die veränderlichen Informationen wie Bildern oder Zeichen aus einer Vielzahl von lichtemittierenden Leuchtflächen erzeugt, die vorzugsweise auch unterschiedliche Farben erzeugen können.

Unter einer Oberschicht, die "im Wesentlichen Metall und/oder ein undurchsichtiges und/oder nicht-lichtreflektierendes Material" umfasst, wird in der vorliegenden Beschreibung vorzugsweise verstanden, dass die Oberschicht ein Bruchverhalten aufweist, das vorzugsweise einem Metall oder einem Kunststoff nahekommt, oder dass die Oberschicht ein duktiles Verhalten, insbesondere eines Kunststoffes oder eines Metalls, aufweist. Insbesondere wird unter einer Oberschicht, die "im Wesentlichen Metall und/oder ein undurchsichtiges und/oder nicht-lichtreflektierendes Material" aufweist, vorzugsweise eine Schicht verstanden, die Metall und/oder ein undurchsichtiges und/oder nicht-lichtreflektierendes Material zu mindestens 2%, insbesondere 5% des Werkstoffs enthält. Je höher der Anteil des Metalls, des undurchsichtigen und/oder des nicht-lichtreflektierenden Materials desto höher die mechanische Stabilität gegen Bruch bzw. desto höher die Bruchfestigkeit der Oberschicht. Daher ist eine Oberschicht, die Metall und/oder ein undurchsichtiges und/oder nicht-lichtreflektierendes Material zu mindestens 90% umfasst, besonders bevorzugt, da eine derartige Ausgestaltung nahezu den Werkstoffeigenschaften eines Metalls, eines undurchsichtigen und/oder nicht-lichtreflektierenden Materials entspricht.

Metall hat den Vorteil, dass dieses - je nach Ausgestaltung - eine hohe Oberflächenfestigkeit gegen äußere Einflüsse, wie Kratzer oder Stöße, aufweist. Auch übersteht es äußere mechanische Einflüsse, wie Stürze, dank einer hohen Elastizitätsgrenze oder dank plastischer Verformung.

Vorteilhafterweise handelt es sich bei dem undurchsichtigen Material um einen Kunststoff, insbesondere um einen duktilen Kunststoff, der sich erst elastisch verformt und erst nach plastischer Verformung bricht. Auf diese Weise kann die Anzeigevorrichtung bestmöglich gegen äußere mechanische Einflüsse, wie Stürze, gesichert werden. Auch ist es günstig, wenn das undurchsichtige Material mechanische Einflüsse mit einer hohen Elastizitätsgrenze kompensieren kann.

Des Weiteren ist es günstig, wenn es sich bei dem nicht-lichtreflektierenden Material um einen Werkstoff, insbesondere einen Kunststoff, handelt, der zudem günstigerweise duktile Eigenschaften aufweist, um eine elastische Dehnung/Verformung zu zulassen und erst nach einer plastischen Verformung zu brechen. Auch hat das nicht-lichtreflektierende Material den Vorteil, dass einfallendes Licht keine störenden Lichtreflexionen erzeugt, die den Betrachter einer Anzeigevorrichtung beim Betrachten beeinträchtigen. Des Weiteren ist es bei einem weiteren Verfahrensschritt vorteilhaft, dass in der Oberschicht Mikro-Durchgänge zum Durchlassen von erzeugtem Licht der lichtemittierenden Leuchtflächen des Displays gebildet werden, die in der zu einem Betrachter gewandten Oberfläche Mikro-Öffnungen bilden. Somit kann Licht von lichtemittierenden Leuchtflächen eines Displays durch die Oberschicht bzw. durch die Mikro-Durchgänge hindurchtreten und an den Mikro-Öffnungen der zu einem Betrachter gewandten Oberfläche der Anzeigevorrichtung austreten. Dabei umfasst die Oberschicht - wie bereits erwähnt - ein vorzugsweise nicht-transparentes Material, wie z. B. Metall. Mithilfe der Ausgestaltung der Oberschicht als - mit anderen Worten ausgedrückt - perforierte Schicht ist es möglich, einen verbessertem Kontrast zu schaffen, insbesondere einen verbesserten Schwarz-Kontrast. Bei Kombination der Mikro-Durchgänge mit der Oberschicht, die vorzugsweise im Wesentlichen Metall oder ein undurchsichtiges oder ein nicht-lichtreflektierendes Material aufweist, wird also ferner eine verstärkte, dünnere Anzeigevorrichtung mit verbessertem Kontrast geschaffen.

Vorzugsweise werden die Mikro-Öffnungen an der zu einem Betrachter gewandten Oberfläche der Oberschicht mit einem Anteil von weniger als 10% an der gesamten zum Betrachter gewandten Oberfläche der Oberschicht eingebracht. Mit andere Worten ausgedrückt, ist es bevorzugt, dass in die Oberschicht Micro-Durchgänge eingebracht werden, die ein Durchtreten von Licht erlauben, das auf der einen Seite der Oberschicht erzeugt wird und auf der anderen Seite der Oberschicht abgegeben wird. Dabei tritt vorzugsweise das erzeugte Licht aus den Mikro-Öffnungen in der zu einem Betrachter gewandten Oberfläche der Oberschicht aus und kann somit zu einem Betrachter gelangen. Aufgrund des bevorzugten" Umstandes, dass der Anteil der Mikro-Öffnungen an der gesamten zum Betrachter gewandten Oberfläche der Oberschicht weniger als 10% beträgt, ist eine im Wesentlichen bzw. weitgehend geschlossene Oberfläche einer Anzeigevorrichtung gewährleistbar, die vorzugsweise ein hohes Maß an Bruchfestigkeit aufweist, wenn sie mit einem duktilen Werkstoff wie z. B. Metall realisiert wird. Auch kann mithilfe der genannten Ausgestaltung ein hoher Kontrastumfang bzw. ein hoher Kontrastwert/Kontrast erzeugt werden.

Bevorzugt sind die Abstände zwischen zwei Mikro-Öffnungen kleiner als das Auflösungsvermögen des Betrachters. Dabei handelt es sich um solche Abstände, die bei dem Betrachter unter einem Winkel von maximal zwei Winkelminuten erscheinen. Hierdurch sind Pixel bzw. Mikro-Öffnungen für einen Großteil der Betrachter nicht mehr unterscheidbar, da das menschliche Auflösungsvermögen in ungünstigen Fällen in etwa zwei Winkelminuten entspricht.

Besonders bevorzugt erscheinen die Abstände der Mikro-Öffnungen dem Betrachter maximal unter einem Winkel von 1 Winkelminute, besonders bevorzugt 0,5 Winkelminuten, besonders bevorzugt 0,25 Winkelminuten. Hierdurch sind für nahezu alle menschlichen Betrachter Pixel bzw. Mikro-Öffnungen nicht mehr unterscheidbar. Je kleiner der maximale Winkel, desto weniger Betrachter existieren, deren Sehfähigkeit zur Unterscheidung zweier Pixel bzw. Mikro-Öffnungen oder Leuchtflächen ausreicht.

Bevorzugt weist das oder weisen die Augen des Betrachters einen Abstand zu der Anzeigefläche auf, welche für die konkrete Art der Anzeigevorrichtung bei der Benutzung der Anzeigevorrichtung üblich ist. Zum Beispiel beträgt der Abstand 5 cm bis 1,20 m, bevorzugt 15 cm bis 60 cm für Displays von Handheldgeräten (Mobiltelefon, Uhr, Tablet-Computer), 25 cm bis 2 m, bevorzugt 40 cm bis 1 m für Displays von Desktop-Computer, 1 m bis 7 m bevorzugt 2 m bis 5 m für Fernsehgeräte und/oder 2 m bis 100 m, bevorzugt 5 m bis mehr als 100 m für Werbe-/Informationsanzeigetafeln. Besonders bevorzugt sind die Abstände, die kleiner als das Auflösungsvermögen des Betrachters sind, z. B. kleiner als 30 µm (800 bis 1000 DPI (Dot Per Inch) für kleine Geräte oder z.B. ein Abstand im Falle eines Fernsehers kleiner als 0,5 mm, ist, bei dem der Benutzer in üblicher Betrachtungsentfernung (> 2m) die einzelnen Pixel bzw. Mikro-Öffnungen oder Leuchtflächen dann nicht mehr voneinander unterscheiden kann.

Z. B. ist die Anzeigevorrichtung ein Desktop-Computer mit einer minimalen Nutzerdistanz (bezogen auf das Auge) von 50 cm. In der hypothetischen Annahme, dass z.B. 70% der potentiellen Benutzer über kein besseres Auflösungsvermögen als 0,6 Winkelminuten verfügen, werden die Pixel bzw. Mikro-Öffnungen oder Leuchtflächen der Anzeigevorrichtung kleiner gleich 87 µm, bevorzugt 80 µm für einen Sicherheitspuffer, voneinander beabstandet, damit für zumindest 70% der Benutzer bei der üblichen Benutzung der Anzeigevorrichtung eine besonders hochwertige Darstellung ermöglicht wird, da für sie einzelne Leuchtflächen bzw. Mikro-Öffnungen nicht unterscheidbar sind. Es resultiert eine Auflösung der Anzeigevorrichtung von 317 DPI, bevorzugt 320 DPI mit Sicherheitspuffer.

Bevorzugt betragen die Abstände, die kleiner als das Auflösungsvermögen des Betrachters sind, maximal 190 µm, bevorzugt maximal 80 µm, besonders bevorzugt maximal 50 µm. Hierdurch wird erreicht, dass, auch wenn der Betrachter der Anzeigefläche näher als üblich kommt, er einzelne Pixel oder Mikro-Öffnungen bzw. Leuchtflächen voneinander nicht unterscheiden kann. Je nach Auflösungsvermögen und Akkommodationsfähigkeit des individuellen Betrachters ist es damit sogar egal wie nahe der individuelle Betrachter der Anzeigevorrichtung kommt, denn er kann selbst bei optimaler Ausnutzung seiner Sehkraft und einem Annähern bis an die Naheinstellgrenze seines Auges kaum die einzelnen Pixel bzw. Mikro-Öffnungen oder Leuchtflächen voneinander unterscheiden.

Bevorzugt beträgt die Ausdehnung einer, bevorzugt jeder, Mikro-Öffnungen bzw. Leuchtfläche höchstens 70 µm, bevorzugt höchstens 25 µm, besonders bevorzugt höchstens 10 µm oder sogar höchstens 5 µm. Hierdurch wird erreicht, dass der Betrachter Mikro-Öffnungen bzw. Leuchtflächen, mit bloßem Auge (in ausgeschaltetem Zustand) nicht erkennen kann und der Kontrast höher wirkt. Die Ausdehnung ist bevorzugt eine maximale Ausdehnung einer Mikro-Öffnung bzw. einer Leuchtfläche parallel zur Anzeigefläche. Besonders bevorzugt ist die Ausdehnung kleiner oder gleich der Wellenlänge des sichtbaren und/oder des durchzuleitenden Lichts. Sie beträgt z.B. höchstens 2 µm, bevorzugt höchstens 1 oder höchstens 0,5 µm. Hierdurch ist insbesondere aufgrund des Rayleigh-Kriteriums der Austrittswinkel des von der Leuchtfläche ausgehenden Lichts größer (und deshalb vorteilhaft, da man die Anzeigevorrichtung aus größeren Winkeln betrachten kann).

Unter einer-Mikro-Öffnung wird in dieser Beschreibung vorzugsweise auch ein Teilbereich in z. B. der Oberschicht verstanden, der bzw. die das Austreten bzw. Hindurchtreten von Licht gestattet. Günstigerweise ist eine Mikro-Öffnung Teil eines Mikro-Durchganges bzw. am Anfang und/oder am Ende des Mikro-Durchganges angeordnet.

Des Weiteren ist es von Vorteil, wenn das Display wenigstens ein VCSEL oder wenigstens ein OLED oder wenigstens ein LED oder ein Mikro-Display umfasst. Dies erlaubt eine einfache Realisation von darzustellenden Lichtinformationen mittels lichtemittierender Leuchtflächen. Auch ist es von Vorteil, wenn lichtemittierende Leuchtfläche in Ausgestaltung eines Display-Chips, vorzugsweise eines LED-Displays, und/oder eines OLED-Displays und/oder eines Plasma-Displays und/oder eines FED-Displays und/oder eines SED-Displays und/oder eines LCD-Displays und/oder eines Lasers und/oder eines VCSEL-Displays realisiert sind. Selbstverständlich ist es auch möglich, dass die Licht emittierenden Leuchtflächen nicht ganze Displays aufweisen sondern stattdessen einzelne oder mehrere Display-Chips, Mikro-Displays, LEDs, OLEDs, LCDs, Laser und/oder VCSEL, insbesondere in Kombination mit einem Quantenpunkt (Quantum Dot).

Vorzugsweise wird das wenigstens eine VCSEL zumindest teilweise innerhalb eines Mikro-Durchganges eingebracht. Auf diese Weise kann das von einem VCSEL erzeugte Licht idealerweise vollständig aus einer Mikro-Öffnung an der zu einem Betrachter gewandten Oberfläche treten. Dadurch, dass das erzeugte Licht nahezu vollständig aus der Anzeigevorrichtung austritt, werden Verluste reduzierbar, wodurch die Energieaufnahme ebenfalls reduziert wird. Ferner erlaubt das Anordnen bzw. zumindest teilweise Einbringen wenigstens eines VCSELs in einem Mikro-Durchgang der Oberschicht, eine Reduzierung der Bauhöhe der gesamten Anzeigevorrichtung.

Wie bereits angedeutet ist es von Vorteil, wenn eine lichtemittierende Leuchtfläche zum Beispiel eine oder mehrere VCSEL bzw. VCSEL Laser mit z.B. 5 µm Strahldurchmesser aufweist, die auf einem Substrat (wie zum Beispiel einem Si-Waver) als Array bzw. in einer Matrix angeordnet sind, z.B. in Form eines Quadrats mit z.B. 50 µm Kantenlänge. Die VCSEL-Laser bzw. VCSEL in einem Quadrat weisen bevorzugt voneinander verschiedene Emissionswellenlängen auf oder, wenn sie die gleiche Emissionswellenlänge aufweisen, werden durch Wellenlänge-Einrichtungen (wie z. B. Farb-Phosphor-Elemente oder Quantenpunkte) in unterschiedliche Emissionswellenlängen konvertiert. Viele solcher Einheiten bilden dann Pixel oder Subpixel der lichtemittierenden Leuchtflächen der Anzeigevorrichtung.

Des Weiteren ist es von Vorteil, wenn das wenigstens eine OLED oder das wenigstens eine LED oder das Mikro-Display außerhalb eines Mikro-Durchganges angeordnet werden. Auf diese Weise ist es möglich, dass das wenigstens eine OLED oder das wenigstens eine LED oder das Mikro-Display beispielsweise unterhalb der Oberschicht angeordnet ist, sodass z. B. mehrere OLEDs, LEDs oder mehrere lichterzeugende Pixel eines Micro-Displays Licht für einen Mikro-Durchgang erzeugen können.

Ferner ist es günstig, wenn die Oberschicht mittels eines generativen Fertigungsverfahrens aufgebaut wird bzw. wenn ein Aufbauen der Oberschicht mittels eines generativen Fertigungsverfahrens erfolgt.

Dabei handelt es sich vorteilhafterweise um selektives Laserschmelzen (SLM), selektives Lasersintern (SLS), Selective Head Sintering (SHS), Binder Jetting, Elektronenstrahlschmelzen (EBM), Fused Deposition Modeling (FDM), Laminated Object Modelling (LOM), Cladding, Wax Deposition Modeling (WDM), Contour Crafting, Kaltgasspritzen, Stereolithografie (SLA), Digital Light Processing (DLP) und/oder Liquid Composite Moulding (LCM).

Bevorzugterweise umfasst ein Aufbauen der Oberschicht ein Erstellen wenigstens einer Schicht und/oder ein Erstellen wenigstens einer Funktionsschicht und/oder ein Erstellen mindestens einer Strahlformeinrichtung. Dabei kann die mindestens eine Strahlformeinrichtung einen Mikro-Durchgang bilden. Auch ist es möglich, mittels eines Lasers und/oder mittels einer spanabhebenden Bearbeitung, vorzugsweise mittels Fräsen und/oder Bohren, und/oder mittels eines chemischen Verfahrens, vorzugsweise mittels Ätzens, und/oder mittels Polierens und/oder Schleifens einen Mikro-Durchgang zu erzeugen. Auch kann beim Aufbauen ein Träger aufgebaut werden, der das Erstellen und/oder Aufbauen weiterer Strukturen, Schichten und/oder Strahlformeinrichtungen auf einfache Weise ermöglicht.

Ferner ist es möglich, dass eine Vielzahl von Strahlformeinrichtungen in einer Matrix angeordnet ist. Somit fungiert die Vielzahl von Strahlformeinrichtungen als Pixel bzw. Bildpunkte, die in Summe ein Bild zusammensetzen können.

Dabei ist es von Vorteil, wenn die Oberschicht mittels eines generativen Fertigungsverfahrens aufgebaut wird und mindestens eine Strahlformeinrichtung umfasst, welche einen Mikro-Durchgang bildet. Somit kann Licht durch die mindestens eine Strahlformeinrichtung und auch durch die Oberschicht hindurchtreten.

Vorzugsweise wird als wenigstens eine Funktionsschicht eine Solarschicht zur Gewinnung von Energie erstellt. Dies erlaubt eine Ausgestaltung der Anzeigevorrichtung ohne Energiespeicher oder mit einem relativ kleinen Energiespeicher, der ebenfalls von der Solarschicht geladen werden kann.

Ferner ist es bevorzugt, dass eine berührungsempfindliche Schicht zur Erfassung von Eingaben als wenigstens eine Funktionsschicht erstellt wird. Auf diese Weise können Eingaben eines Betrachters bzw. Benutzers über die Anzeigevorrichtung erfasst werden, wodurch die Anzeigevorrichtung als Eingabegerät bei z. B. Smartphones, Smartwatches oder Tablets eingesetzt werden kann.

Vorzugsweise wird eine druckempfindliche Schicht zur Erfassung von Druck als wenigstens eine Funktionsschicht erstellt. Auch diese Ausgestaltung erlaubt es, dass Eingaben eines Betrachters bzw. Benutzers über die Anzeigevorrichtung erfasst werden. Dadurch kann die Anzeigevorrichtung als Eingabegerät auch bei z. B. Smartphones, Smartwatches oder Tablets eingesetzt werden. Ferner kann mithilfe einer druckempfindlichen Schicht beispielsweise der Puls eines Smartwatchträgers ermittelt werden.

Günstigerweise wird eine temperaturempfindliche Schicht zur Messung einer Temperatur als wenigstens eine Funktionsschicht erstellt. Dies gestattet es der Anzeigevorrichtung die äußere Temperatur und/oder z. B. die Temperatur des Trägers einer Smartwatch zu erfassen.

Ferner kann vorgesehen sein, dass eine kapazitive Schicht zur Messung einer Kapazität als wenigstens eine Funktionsschicht erstellt wird. Somit können Eingaben eines Betrachters bzw. Benutzers über eine derartige Schicht der Anzeigevorrichtung erfasst werden, wodurch die Anzeigevorrichtung bei z. B. Smartphones, Smartwatches oder Tablets eingesetzt als Eingabegerät verwendet werden kann.

Des Weiteren ist es günstig, wenn beim Erstellen der wenigstens einen Funktionsschicht mindestens ein sensorisches Element in die Funktionsschicht eingebracht wird. Auf diese Weise kann beispielsweise ein Sensorchip in eine Funktionsschicht der Oberflächenschicht eingebracht werden, um diese Schicht zur Erfassung von z. B. einer Temperatur auszugestalten.

Auch ist es von Vorteil, wenn das mindestens eine sensorische Element als Touchsensor und/oder als Temperatursensor und/oder als Drucksensor und/oder als kapazitiver Sensor ausgebildet ist. Auf diese Weise wird es der Anzeigevorrichtung möglich, Eingaben eines Betrachters bzw. Benutzers zu erfassen, wodurch die Anzeigevorrichtung bei z. B. Smartphones, Smartwatches oder Tablets eingesetzt werden kann. Ebenfalls kann die äußere Temperatur und/oder z. B. die Temperatur des Trägers einer Smartwatch erfasst werden.

Idealerweise umfasst ein Aufbauen der Oberschicht ein Erstellen einer oberflächenbildenden Oberflächenschicht bzw. einer äußeren Oberfläche, die die zu einem Betrachter gewandte Oberfläche bildet. Die oberflächenbildende Oberflächenschicht bzw. die äußere Oberfläche bildet günstigerweise den Bereich der zu einem Betrachter gewandten Oberfläche, der von dem Betrachter physisch berührbar ist. D.h., dass dieser Bereich bzw. diese Oberflächenschicht bzw. die Oberfläche mit Fingern, Hand und somit Haut eines Betrachters in Berührung gelangt. Dementsprechend können Qualitätsmerkmale bzw. eine bestimmte Oberflächenrauigkeit an die Oberflächenschicht gestellt werden, die die Haptik einer Anzeigevorrichtung angenehm empfinden lassen.

Bei einer weiteren vorteilhaften Ausführung umfasst das Erstellen der Oberflächenschicht ein Einbringen eines Edelmetalls in die Oberflächenschicht bzw. in die äußere Oberfläche zur Veredelung. Auf diese Weise können beispielsweise inerte Materialien in die zu einem Betrachter gewandte Oberfläche eingebracht werden. Unter einem inerten Material wird vorteilhafterweise in diesem Zusammenhang von Material, wie z. B. Gold oder Silber, verstanden, die unter den jeweilig gegebenen Bedingungen mit potentiellen Reaktionspartnern (etwa Luft, Wasser, Edukte und Produkte einer Reaktion) nicht oder nur in verschwindend geringem Maße reagieren.

Vorzugsweise bildet die Oberflächenschicht auch einen Schutz für die Oberschicht und/oder erhöht die Wiederstandfähigkeit der Oberschicht und/oder bildet eine Verschönerung für die Oberschicht, beispielsweise mittels eines lichtdurchlässigen Kunststoffs. Somit kann ein ansprechendes Äußeres mit funktionellen Eigenschaften verknüpft werden.

Günstigerweise umfasst das Erstellen der Oberflächenschicht bzw. der äußeren Oberfläche, ein Aufbringen einer Oberflächenstruktur. Auf diese Weise kann die Haptik der Anzeigevorrichtung verbessert werden, insbesondere können auch Eigenschaften wie eine fettabweisende Oberflächenstruktur ähnlich dem Lotoseffekt realisiert werden.

Vorzugsweise wird die Oberflächenstruktur hydrophob und/oder oleophob und/oder bakteriophob und/oder lichtdurchlässig ausgebildet. Auf diese Weise kann die Oberflächenschicht bzw. die Oberschicht der Anzeigevorrichtung bzw. die äußere Oberfläche abweisend für Wasser, Öl und/oder Bakterien ausgebildet werden. Ferner dient die lichtdurchlässige Ausbildung dem Durchlassen von Licht von der Anzeigevorrichtung bzw. deren lichtemittierenden Leuchtflächen bzw. der Oberschicht. Selbstverständlich kann die Oberflächenstruktur auch so ausgebildet werden bzw. sein, dass auch auf die Anzeigevorrichtung bzw. die Oberschicht einfallendes Licht von der Oberflächenstruktur durchgelassen wird. Bevorzugterweise wird beim Erstellen der mindestens einen Strahlformeinrichtung die mindestens eine Strahlformeinrichtung zumindest teilweise innerhalb der Oberschicht erstellt. Somit kann die mindestens eine Strahlformeinrichtung also innerhalb, außerhalb oder zumindest teilweise innerhalb und teilweise außerhalb angeordnet sein. Jede Anordnung bringt unterschiedliche Vorteile mit sich. So ist z. B. eine Anordnung der mindestens einen Strahlformeinrichtung innerhalb der Oberschicht sinnvoll, wenn die Oberschicht der Anzeigevorrichtung auf ein OLED aufgebracht wird. Auf diese Weise können die Oberschicht und das OLED unabhängig voneinander hergestellt werden. Beispielsweise ist eine Anordnung der mindestens einen Strahlformeinrichtung außerhalb der Oberschicht wünschenswert, wenn die Oberschicht der Anzeigevorrichtung mit einem VCSEL kombiniert wird. Denn mit dieser Ausgestaltung kann der Raumbedarf reduziert werden und eine sehr dünne Anzeigevorrichtung realisiert werden.

Vorzugsweise bildet die mindestens eine Strahlformeinrichtung einen Mikro-Durchgang. Unter einem Mikro-Durchgang wird vorzugsweise eine Verbindung zweier Seiten einer Schicht, insbesondere der Oberschicht, verstanden. Anders ausgedrückt, ist es von Vorteil, wenn mindestens eine Mikro-Durchgang bildende Strahlformeinrichtung ähnlich einer Bohrung durch ein Blech ausgebildet ist. So kann also ein Mikro-Durchgang bzw. mindestens eine Strahlformeinrichtung ein Hohlraum innerhalb einer Schicht, insbesondere der Oberschicht, sein. Die Form des Hohlraums bzw. des Mikro-Durchgangs/Strahlformeinrichtung kann dabei beliebig ausgestaltet sein, jedoch ist eine zylindrische Form bevorzugt. Mit anderen Worten ausgedrückt, kann es vorteilhaft sein, die mindestens eine Strahlformeinrichtung als Hohlraum in einer Schicht auszubilden, wobei der Hohlraum ähnlich einem Bohrloch bzw. ähnlich einem Durchgangsloch ausgebildet ist, wobei der Hohlraum mit einem Gas, insbesondere Luft, gefüllt sein kann.

Des Weiteren ist es günstig, wenn die mindestens eine Strahlformeinrichtung zumindest teilweise aus einem lichtdurchlässigen und/oder elektrisch leitenden Material, insbesondere Kunststoff, erstellt wird. Ein zumindest teilweise lichtdurchlässiges Material erlaubt z. B. die Weiter- bzw. Durchleitung von Licht, das beispielsweise von einem OLED oder LED erzeugt wird, durch die Oberschicht, wobei vorzugsweise der Verlust an Lichtleistung vermindert wird. Auch kann es sich bei dem zumindest teilweise lichtdurchlässigen Material um eine Glasfaser, insbesondere aus einer "Photonic Crystal Fiber" (PCF) oder auch "Photonic Bandgap Fiber" (PBG-Fiber), handeln. Diese Glasfaser / dieses Material leitet Licht nahezu verlustfrei, wobei deren Eigenschaften nahezu beliebig einstellbar sind. Mittels eines elektrisch leitenden Materials können weitere Eigenschaften der Anzeigevorrichtung verbessert werden, insbesondere die Fähigkeit Touch-Eingaben bzw. berührungssensitive Eingaben mit hoher Genauigkeit zu erfassen.

Auch ist es günstig, wenn die mindestens eine Strahlformeinrichtung zumindest an ihrer zu einem Betrachter gewandten Oberfläche bzw. Seite ein elektrisch leitendes Material und/oder ein undurchsichtiges und/oder ein nicht-reflektierendes Material umfasst. Günstigerweise kann durch die mindestens eine Strahlformeinrichtung, die zumindest an ihrer zu einem Betrachter gewandten Oberfläche ein elektrisch leitendes Material umfasst, die Oberfläche und/oder der Zwischenraum zwischen mindestens zwei Strahlformeinrichtungen angepasst werden, den Empfang von Funksignalen für Antennen zu verbessern. Denn das elektrisch leitende Material kann sowohl für den Empfang von Funksignalen aber auch für das Senden solcher genutzt werden.

Ferner ist es bevorzugt, dass die mindestens eine Strahlformeinrichtung ein Streuelement, insbesondere einen Diffusor, und/oder einen Kollimator und/oder einen Konzentrator, aufweist. Anders ausgedrückt wird bevorzugterweise die mindestens eine Strahlformeinrichtung mit einem Streuelement, insbesondere einem Diffusor und/oder einem Kollimator und/oder einem Konzentrator ausgebildet bzw. hergestellt. Dabei kann ein Streuelement bzw. ein Diffusor Licht in einem bestimmten Winkelbereich abgeben bzw. Licht streuen. Dadurch kann der Blickwinkel, unter welchem Lichtinformation für einen Betrachter erkennbar ist, eingestellt werden. Des Weiteren kann mit einem Kollimator ein paralleler Strahlenverlauf erzeugt werden, wodurch Licht von einer lichtemittierenden Leuchtfläche, wie z. B. einem LED oder OLED, konzentriert in eine vorgegebene Richtung abgegeben bzw. gerichtet werden kann. Mithilfe des Konzentrators ist es möglich, das von einer lichtemittierenden Leuchtfläche, insbesondere von einem LED, OLED oder dergleichen, erzeugte bzw. emittierte Licht zu konzentrieren. Dadurch kann die Lichtausbeute erhöht und somit zum einen die Lichtstärke aber auch die Lichtintensität gesteigert werden, und zum anderen Energie eingespart werden, sodass eine Anzeigevorrichtung mit einem Konzentrator eine hohe Energieeffizienz aufweist.

Auch kann vorgesehen sein, dass die mindestens eine Strahlformeinrichtung einen Ein- und einen Ausgang für Licht umfasst. Auf diese Weise kann Licht vom Eingang zum Ausgang gelangen.

Vorzugsweise wird der Eingang an zumindest einer lichtemittierenden Leuchtfläche des Displays angeordnet, wobei günstigerweise der Ausgang an der zu einem Betrachter gewandten Oberfläche der Oberschicht angeordnet wird. Somit kann also Licht einer lichtemittierenden Leuchtfläche am Eingang der mindestens einen Strahlformeinrichtung in diese eingeleitet werden, und dieses eingeleitete Licht am Ausgang wieder abgegeben werden. In der bevorzugten Ausgestaltung der mindestens einen Strahlformeinrichtung mit einem Diffusor und/oder einem Kollimator und/oder einem Konzentrator ist es von Vorteil, wenn der Konzentrator am Eingang angeordnet wird, um maximal Licht einer lichterzeugenden Leuchtfläche in die mindestens eine Strahlformeinrichtung einzubringen. Vorteilhafterweise wird/ist der Ausgang der mindestens einen Strahlformeinrichtung innerhalb und/oder an der Mikro-Öffnung angeordnet/anordenbar.

Des Weiteren ist es bevorzugt, wenn am Ausgang der mindestens einen Strahlformeinrichtung ein Streuelement, insbesondere ein Diffusor, und/oder ein Farbfilter-Element und/oder ein farbkonvertierendes Element und/oder ein Farb-Phosphor-Element, insbesondere ein Quantenpunkt, positioniert wird. Der Diffusor erlaubt eine Abgabe des Lichts aus der mindestens einen Strahlformeinrichtung in einem vorherbestimmbaren Winkelbereich. Auf diese Weise kann der Blickwinkel auf die jeweiligen Bedürfnisse für eine Anzeigevorrichtung angepasst werden. So kann beispielsweise ein enger Blickwinkel für Anzeigevorrichtungen mit sensiblen Daten geeignet sein, wohingegen ein großer Blickwinkel für z. B. eine Smartwatch von Vorteil ist. Das Farbfilter-Element, das farbkonvertierende Element und/oder das Farb-Phosphor-Element, insbesondere in Ausgestaltung als ein Quantenpunkt, können dazu dienen, Licht einer bestimmten Wellenlänge und somit einer bestimmten Farbe in Licht einer anderen Wellenlänge bzw. Farbe umzuwandeln. Dies ist vorteilhaft bei der Verwendung wenigstens eines VCSEL in der Anzeigevorrichtung. Somit kann also auch mit wenigstens einem VCSEL eine Anzeigevorrichtung geschaffen werden, die unterschiedliche Farben im RGB-Farbraum realisiert.

Auch ist es von Vorteil, wenn der Ausgang der mindestens einen Strahlformeinrichtung ausgebildet ist, Licht für eine Farb- und/oder Strahlungsmessung zu erfassen und/oder auszuwerten. Auf diese Weise kann also nicht nur Licht vom Ausgang abgegeben werden, sondern auch vom Ausgang aufgenommen werden. Somit kann zum Beispiel eine Lichtinformation oder elektromagnetische Strahlungsinformation von der mindestens einen Strahlformeinrichtung, insbesondere deren Ausgang, hin zum Eingang geleitet werden. In diesem Zusammenhang ist es bevorzugt, wenn am Eingang nicht nur eine lichtemittierende Leuchtfläche, sondern auch ein lichtempfindlicher Sensor angeordnet ist. Dieser kann die Lichtinformation oder elektromagnetische Strahlungsinformation, die vom Ausgang aufgenommen wurde und zum Eingang der mindestens einen Strahlformeinrichtung geleitet wurde, aufnehmen und beispielsweise in ein elektrisches Signal umwandeln. Auf diese Weise ist es zum Beispiel möglich, ein Foto der Umgebung mithilfe einer derartig ausgestalteten Anzeigevorrichtung aufzunehmen oder aber die Anzeigevorrichtung zum Kopieren eines Dokuments einzusetzen. Insbesondere bei der Erfassung und/oder Auswertung von Licht für eine Farbmessung ist es günstig, wenn der erwähnte Sensor für das dementsprechende Lichtspektrum empfindlich ausgebildet ist. Im Zusammenhang mit der Strahlungsmessung ist es von Vorteil, dass nicht nur sichtbares Licht, sondern auch Strahlung, insbesondere UV-Strahlung bzw. auch IR-Strahlung, vom Ausgang der mindestens einen Strahlformeinrichtung hin zum Eingang, insbesondere zur Licht emittierenden Leuchtfläche bzw. einem dort angeordneten Sensor, empfindlich für genannte Strahlungsarten, geleitet wird.

Auch ist es günstig, wenn die mindestens eine Strahlformeinrichtung angepasst ist, Lichtinformation in beide Richtungen zu leiten, insbesondere Licht vom Eingang zum Ausgang und vom Ausgang zum Eingang der mindestens einen Strahlformeinrichtung. Somit kann also nicht nur Licht vom Ausgang abgegeben werden, sondern auch vom Ausgang aufgenommen werden. Dadurch kann zum Beispiel eine Lichtinformation von der mindestens einen Strahlformeinrichtung, insbesondere deren Ausgang, hin zum Eingang geleitet werden. In diesem Zusammenhang ist es bevorzugt, wenn am Eingang nicht nur eine lichtemittierende Leuchtfläche, sondern auch ein lichtempfindlicher Sensor angeordnet ist. Dieser kann die Lichtinformation, die vom Ausgang aufgenommen wurde und zum Eingang der mindestens einen Strahlformeinrichtung geleitet wurde, aufnehmen und beispielsweise in ein elektrisches Signal umwandeln. Auf diese Weise ist es zum Beispiel möglich, ein Foto der Umgebung mithilfe einer derartig ausgestalteten Anzeigevorrichtung aufzunehmen oder aber die Anzeigevorrichtung zum Kopieren eines Dokuments einzusetzen. Insbesondere bei der Erfassung und oder Auswertung von Licht für eine Farbmessung ist es günstig, wenn der erwähnte Sensor für das dementsprechende Lichtspektrum empfindlich ausgebildet ist. Im Zusammenhang mit der Strahlungsmessung ist es von Vorteil, dass nicht nur sichtbares Licht, sondern auch Strahlung, insbesondere UV-Strahlung bzw. auch IR-Strahlung, vom Ausgang der mindestens einen Strahlformeinrichtung hin zum Eingang, insbesondere zur Licht emittierenden Leuchtfläche bzw. einem dort angeordneten Sensor, empfindlich für genannte Strahlungsarten, geleitet wird.

Des Weiteren umfasst die mindestens eine Strahlformeinrichtung ausgangsseitig ein Opferelement. Anders ausgedrückt ist in einem Verfahrensschritt am Ausgang der mindestens einen Strahlformeinrichtung ein Opferelement angeordnet. Dieses ist vorzugsweise zylindrisch, quaderförmig oder kegelförmig und vorzugsweise lichtdurchlässig ausgebildet. Ferner ist es von Vorteil, wenn das Opferelement Licht aufnimmt und weiterleitet. Das Opferelement dient erfindungsgemäß, wie der Name schon sagt, als ein Element, das geopfert werden kann. Hierbei kann das Opferelement insbesondere im Zusammenhang mit einem anschließenden Bearbeitungsschritt abgetragen werden, um eine im Wesentlichen ebene oberflächenbildende Oberflächenschicht bzw. eine im Wesentlichen ebene äußere Oberfläche, beispielsweise mittels eines abtragenden Verfahrens, wie zum Beispiel einem Schleifen oder Polieren, zu bilden. So kann mithilfe des Opferelements eine Beschädigung der weiteren Strahlformeinrichtung verhindert werden, da das Opferelement zum Erstellen einer im Wesentlichen ebenen Oberfläche ohne Probleme geopfert werden kann, ohne dass die Funktion der mindestens einen Strahlformeinrichtung durch deren mögliche Beschädigung in Mitleidenschaft gezogen wird. Ferner kann es sich bei einem abtragenden Verfahren auch um eine Bearbeitung mittels eines Lasers und/oder mittels einer spanabhebenden Bearbeitung, vorzugsweise mittels Fräsen und/oder Bohren, und/oder mittels eines chemischen Verfahrens, vorzugsweise mittels Ätzens, und/oder mittels Polieren handeln, um eine Oberflächenschicht zu erzeugen.

Unter einer Oberflächenschicht bzw. einer Oberfläche, die "im Wesentlichen eben" ausgestaltet ist, wird in der vorliegenden Beschreibung vorzugsweise einen Ebenheitstoleranz verstanden. Mit anderen Worten beschrieben, wird bevorzugterweise unter einer "im Wesentlichen ebenen" Oberflächenschicht bzw. Oberfläche eine Fläche verstanden, die zwischen zwei idealen Ebenen vom Abstand 0,5 Millimeter liegt.

Auch kann vorgesehen sein, dass beim Erstellen der mindestens einen Strahlformeinrichtung zwischen mindestens zwei Strahlformeinrichtungen mindestens ein sensorisches Element angeordnet wird. Somit kann der Raum zwischen zwei Strahlformeinrichtungen für sensorische Aufgaben genutzt werden.

Ferner ist es günstig, wenn das Display, vorzugsweise dessen lichtemittierende Seite, mindestens ein sensorisches Element aufweist. Auch ist es möglich, dass zwischen dem Display und der Oberschicht mindestens ein sensorisches Element aufgebracht wird. Auf diese Weise ist es also möglich, dass das sensorische Element an unterschiedlichen Positionen der Anzeigevorrichtung angeordnet wird. Dies hat Vorteile insbesondere im Zusammenhang mit dem jeweiligen Herstellungsverfahren. Auch kann auf diese Weise der Raum zwischen mindestens zwei Strahlformeinrichtungen auf ideale Weise genutzt werden.

Ferner ist es bevorzugt, dass zwischen den oder innerhalb der lichtemittierenden Leuchtflächen des Displays mindestens ein sensorisches Element angebracht wird. Auf diese Weise kann das Display zusammen mit sensorischen Elementen aufgebaut werden, wodurch die Herstellung der Anzeigevorrichtung vereinfacht wird. Mithilfe eines oder mehrerer sensorischer Elemente kann das Display z. B. die Farbe von einfallendem Licht detektieren oder die Umgebungshelligkeit gemessen werden, um beispielsweise die Beleuchtungsstärke des Displays anzupassen.

Vorzugsweise weist das mindestens eine sensorische Element einen Sensor auf, insbesondere einen zweidimensionalen und/oder dreidimensionalen Sensor, vorzugsweise einen Bildsensor und/oder einen helligkeitsempfindlichen und/oder einen berührungsempfindlichen und/oder einen druckempfindlichen und/oder einen gasempfindlichen und/oder einen temperaturempfindlich Sensor, insbesondere ein Piezoelement.

In diesem Zusammenhang ist es beispielsweise bevorzugt, dass das mindestens eine sensorische Element als berührungsempfindlicher Sensor zur Erfassung von Eingaben erstellt bzw. auch angeordnet wird. Auf diese Weise können Eingaben eines Betrachters bzw. Benutzers über die Anzeigevorrichtung erfasst werden, wodurch die Anzeigevorrichtung als Eingabegerät bei z. B. Smartphones, Smartwatches oder Tablets eingesetzt werden kann. Ferner ist es günstig, wenn das mindestens eine sensorische Element als druckempfindlicher Sensor zur Erfassung von Druck erstellt bzw. auch angeordnet wird. Auch diese Ausgestaltung erlaubt es, dass Eingaben eines Betrachters bzw. Benutzers über die Anzeigevorrichtung erfasst werden. Dadurch kann die Anzeigevorrichtung als Eingabegerät auch bei z. B. Smartphones, Smartwatches oder Tablets eingesetzt werden. Ferner kann mithilfe eines druckempfindlichen Sensors beispielsweise der Puls eines Smartwatchträgers ermittelt werden. Ferner ist es möglich, dass das mindestens eine sensorische Element als temperaturempfindlicher Sensor zur Messung einer Temperatur erstellt bzw. auch angeordnet wird. Dies gestattet es der Anzeigevorrichtung günstigerweise die äußere Temperatur und/oder z. B. die Temperatur des Trägers einer Smartwatch zu erfassen. Auch kann vorgesehen sein, dass das mindestens eine sensorische Element als kapazitiver Sensor zur Messung einer Kapazität erstellt bzw. auch angeordnet wird. Somit können zum Beispiel Eingaben eines Betrachters bzw. Benutzers über einen derartigen Sensor der Anzeigevorrichtung erfasst werden, wodurch die Anzeigevorrichtung bei z. B. Smartphones, Smartwatches oder Tablets eingesetzt als Eingabegerät verwendet werden kann. Auch ist es von Vorteil, wenn das mindestens eine sensorische Element als Touchsensor und/oder als Temperatursensor und/oder als Drucksensor und/oder als kapazitiver Sensor ausgebildet ist. Auf diese Weise wird es der Anzeigevorrichtung möglich, Eingaben eines Betrachters bzw. Benutzers zu erfassen, wodurch die Anzeigevorrichtung bei z. B. Smartphones, Smartwatches oder Tablets eingesetzt werden kann. Ebenfalls kann die äußere Temperatur und/oder z. B. die Temperatur des Trägers einer Smartwatch erfasst werden. Eine konkrete Ausgestaltung eines berührungsempfindlichen Sensors ist zum Beispiel ein Piezoelement oder aber eine druckresistive Ausbildung der Anzeigevorrichtung. Ein temperaturempfindlicher Sensor kann beispielsweise ein elektrisches oder elektronisches Bauelement sein, das ein elektrisches Signal als Maß für die Temperatur liefert. Hierbei kann es sich beispielsweise um Bauteile handeln, die ihren Widerstand verändern. Nachstehende Elemente sind hierzu in der Lage und werden beispielhaft aufgezählt: Heißleiter (NTC) verringern ihren Widerstand bei Temperaturerhöhung, Kaltleiter (PTC) erhöhen ihren Widerstand bei Temperaturerhöhung, Silizium-Messwiderstände, Keramik-Kaltleiter. Auch kann es sich um Bauteile handeln, die direkt ein verarbeitbares elektrisches Signal liefern. Hierzu zählen zum Beispiel integrierte Halbleiter-Temperatursensoren (Festkörperschaltkreise), welche einen zu ihrer Temperatur proportionalen Strom oder eine zu ihrer Temperatur proportionale Spannung oder ein von ihrer Temperatur abhängiges digitales Signal liefern. Selbstverständlich kann es sich auch um eine Diode oder einen Temperaturfühler mit Schwingquarz als Messelement handeln. Auch sind sogenannte Thermoelemente denkbar, die eine Temperaturdifferenz durch den Seebeck-Effekt in eine elektrische Spannung umwandeln. Des Weiteren können pyroelektrische Materialien eingesetzt werden, wie sie aus beispielsweise einem Pyrometer oder Wärmebildkameras bekannt sind, wobei diese berührungslos arbeiten und die Wärmestrahlung messen. Auch mechanisch arbeitende Temperaturschalter, z. B. Bimetallschalter, die durch Krümmung eines Bimetalles einen Schalter betätigen, sind einsetzbar. Ein gasempfindliche Sensor hat den Vorteil, dass dieser Gase wie beispielsweise Ozon, CO₂ usw. erkennen kann. Dabei kann ein temperaturempfindlicher Sensor zur Erfassung einer Temperatur, z. B. der Umgebungstemperatur und/oder der Temperatur der Anzeigevorrichtung oder eines bestimmten Bauteils dienen. Mithilfe einer 2-dimensionalen und/oder 3-dimensionalen Ausgestaltung von Sensoren ist es auch möglich, einen Sensor nicht nur flächig innerhalb der Oberschicht der Anzeigevorrichtung anzuordnen, sondern auch entlang der Höhe bzw. Dicke der Oberschicht auszubilden. Auf diese Weise kann erheblich Bauraum eingespart, aber auch für weitere Zwecke genutzt werden. Somit sind also hochfunktionale Einheiten bildbar.

Des Weiteren ist es günstig, dass wenigstens eine Funktionsschicht und/oder wenigstens eine Schicht der Oberschicht und/oder mindestens eine Strahlformeinrichtung mittels eines Dünnschicht-Verfahrens erstellt wird. Auf diese Weise sind sehr dünne Schichten und somit eine sehr dünne bzw. auch leichte Anzeigevorrichtung herstellbar.

Ferner ist es von Vorteil, wenn das Erstellen mittels Dünnschicht-Verfahrens ein Sputtern und/oder ein galvanisches Aufbringen und/oder einen Nanoimprint und/oder ein Walzenprägeverfahren und/oder ein Spritzgussverfahren umfasst. Mithilfe der vorgenannten Verfahren sind Strukturen im Mikrometer- und Nanometerbereich realisierbar. Somit ist es also möglich, eine Oberschicht in den genannten Bereichen zu strukturieren und herzustellen. Vorzugsweise umfasst das Erstellen ein Sputtern mit einem Metall, insbesondere mit Aluminium oder Titan oder Gold oder Silber. Größtenteils wird das Sputtern als Teilschritt bei der Sputterdeposition, einer zur Gruppe der PVD-Verfahren gehörenden feinvakuumbasierten Beschichtungstechnik eingesetzt. Hier dient es zum Zerstäuben eines Materials, das sich anschließend auf einem Substrat niederschlägt und eine feste Schicht bildet. Im Bereich der Beschichtungstechnik wird die Sputterdeposition häufig nur als "Sputtern" bezeichnet. Unter galvanischem Aufbringen wird vorzugsweise ein Elektroplattieren (auch Galvanotechnik) bzw. Nickel- Elektroplattieren (nickel electroplating) bzw. die elektrochemische Abscheidung von metallischen Niederschlägen auf Substrate verstanden. Nickel- Elektroplattieren (nickel electroplating) ist eine Galvanisierungstechnik, bei der eine dünne Schicht aus Nickel auf einem Metallgegenstand aufgebracht wird. Die Nickelschicht kann dekorativer Art sein, bietet Korrosionsbeständigkeit, Verschleißfestigkeit, oder kann zum Aufbau verschlissener oder kleiner Teile genutzt werden. Unter dem Begriff Nanoimprint bzw. Nanoprägelithografie wird vorzugsweise ein Nanolithografie-Verfahren zum kostengünstigen Herstellen von Nanostrukturen verstanden. Hierbei wird mittels eines nanostrukturierten Stempels, welcher ein Negativ bildet, ein Positiv "gestempelt". Für das Positiv werden in der Regel Monomere oder Polymere verwendet, die nach dem Prägen ausgehärtet werden. Beim Walzenprägeverfahren wird die mindestens eine Strahlformeinrichtung von beispielsweise einer Walze aus einem Kunststoffmaterial geprägt. Diese erlaubt eine einfache, kostengünstige und schnelle Herstellung von Strahlformeinrichtungen. Das Spritzgussverfahren ist ein Urformverfahren, welches in der Regel bei der Kunststoffverarbeitung eingesetzt wird. Dabei wird mit einer Spritzgießmaschine der jeweilige Werkstoff verflüssigt (plastifiziert) und in ein die Form bildendes Spritzgießwerkzeug unter Druck eingespritzt. Im Werkzeug geht der Werkstoff durch Abkühlung oder eine Vernetzungsreaktion wieder in den festen Zustand über und wird nach dem Öffnen des Werkzeuges als Fertigteil entnommen. Dabei bestimmt das Werkzeug die Form und die Oberflächenstruktur des fertigen Teiles.

Auch ist es von Vorteil, wenn das Erstellen eine chemische oder physikalische Gasphasenabscheidung oder ein Sol-Gel-Verfahren umfasst.

Als physikalische Gasphasenabscheidung (auch physical vapour deposition, kurz PVD, genannt), wird eine Gruppe von vakuumbasierten Beschichtungsverfahren bzw. Dünnschichttechnologien bezeichnet. Anders als bei der chemischen Gasphasenabscheidung wird mithilfe physikalischer Verfahren ein Ausgangsmaterial in die Gasphase überführt. Das gasförmige Material wird anschließend zum zu beschichtenden Substrat geführt, wo es kondensiert und die Zielschicht bildet. Zu den Verfahren der physikalischen Gasphasenabscheidung zählen z. B. thermisches Verdampfen, Elektronenstrahlverdampfen, Laserstrahlverdampfen, Lichtbogenverdampfen, Molekularstrahlepitaxie, Sputtern oder Ionenplattieren. Dabei können mithilfe der genannten Verfahren der Gasphasenabscheidung nahezu alle Metalle und auch Kohlenstoff abgeschieden werden. Bei Zuführung von Reaktivgasen wie Sauerstoff, Stickstoff oder Kohlenwasserstoffe, sind auch Oxide, Nitride oder Carbide abscheidbar bzw. herstellbar. Die physikalische Gasphasenabscheidung wird in der Regel zur Herstellung dünner Schichten im Bereich einiger Nanometer bis hin zu wenigen Mikrometern eingesetzt.

Als chemische Gasphasenabscheidung (englisch chemical vapour deposition, CVD) wird eine Gruppe von Beschichtungsverfahren zur Herstellung mikroelektronischer Bauelemente und Lichtwellenleiter bezeichnet. Dabei wird eine Oberfläche eines Substrats erhitzt, wodurch aufgrund einer chemischen Reaktion aus der Gasphase eine Feststoffkomponente abgeschieden wird. Das Verfahren der chemischen Gasphasenabscheidung zeichnet sich durch mindestens eine Reaktion an der Oberfläche des zu beschichtenden Werkstücks aus. An der Reaktion müssen wenigstens eine gasförmige Ausgangsverbindung sowie wenigstens zwei Reaktionsprodukte - wovon wenigstens eines in der festen Phase vorliegt - beteiligt sein. Eine Besonderheit dieses Verfahrens ist die gleichmäßige Schichtabscheidung. Im Unterschied zu physikalischen Verfahren ermöglicht die chemische Gasphasenabscheidung auch die Beschichtung von komplex dreidimensional geformten Oberflächen.

Als Sol-Gel-Prozess wird ein Verfahren zur Herstellung nichtmetallischer anorganischer oder hybridpolymerer Materialien aus kolloidalen Dispersionen, den sogenannten Solen bzw. Lösungen bezeichnet. Aus Ausgangsmaterialien werden in Lösung in ersten Grundreaktionen kleinste Teilchen erzeugt. Durch eine besondere Weiterverarbeitung sind Pulver, Fasern, Schichten oder sogar Aerogele erzeugbar.

Des Weiteren ist es bevorzugt, dass das Erstellen der wenigstens einen Funktionsschicht und/oder der wenigstens einen Schicht der Oberschicht und/oder der mindestens einen Strahlformeinrichtung ein Gießen von einem Werkstoff umfasst. Im Unterschied zum Spritzgießen wird beim Gießen der Werkstoff vorzugsweise nicht unter Druck in eine Form eingespritzt, sondern - ähnlich dem Gießvorgang von großen Bauteilen für z. B. Motoren - eine Gießmasse in eine Form gegossen oder auf beispielsweise eine Schicht aufgegossen. Auf diese Weise ist eine im Wesentlichen ebene oberflächenbildende Oberflächenschicht bzw. eine ebene äußere Oberfläche einfach und kostengünstig erzeugbar.

Ferner ist bevorzugt, dass der gegossene Werkstoff mittels UV-Licht, zugeführter oder abgeführter Wärme ausgehärtet wird. Dies erlaubt es der Gussmasse zunächst in die Kavitäten der Form einfließen zu lassen und somit Lufteinschlüsse durch eine Zeitverzögerung nach dem Gießen des Werkstoffes zu verhindern. Auch ist es möglich, vor dem Härten mittels Licht oder Wärme die gegossene bzw. vergossene Masse Vibrationen auszusetzten, die Lufteinschlüsse nach außen befördern können. Auf diese Weise können Lufteinschlüsse - wie bereits angedeutet - verhindert und eine hochwertige Schicht erzeugt werden.

Günstigerweise umfasst das Erstellen der wenigstens einen Funktionsschicht und/oder der wenigstens einen Schicht der Oberschicht und/oder der mindestens einen Strahlformeinrichtung ein Auftragen mittels eines Verspachtel-Vorganges. Bei einem solchen Vorgang wird - vereinfacht ausgedrückt - eine Spachtelmasse mittels eines Spachtels aufgetragen, um eine Oberschicht bzw. einen Teil der Oberschicht, insbesondere die oberflächenbildende Oberflächenschicht bzw. die äußere Oberfläche, zu bilden. Vorteilhaft an diesem Vorgang ist das einfache Aufbringen und Verteilen einer Spachtelmasse.

Bei dem Verspachtel-Vorgang ist es günstig, wenn eine Spachtelmasse in die Mikro-Öffnungen, und vorzugsweise zumindest zum Teil in die Mikro-Durchgänge, eindringt, um mindestens eine Strahlformeinrichtung, insbesondere einen Diffusor, zu bilden. Auf diese Weise kann die Spachtelmasse nicht nur einen Teil der Oberschicht bilden, insbesondere die oberflächenbildende Oberflächenschicht bzw. die äußere Oberfläche, sondern auch gleichzeitig mindestens eine Strahlformeinrichtung, vorteilhafterweise in Ausgestaltung als Diffusor.

Bevorzugterweise umfasst die Spachtelmasse ein Quantenpunkt bildendes Halbleitermaterial. Somit kann also mithilfe der Spachtelmasse nicht nur ein Diffusor, sondern auch ein Quantenpunkt erzeugt werden, mit dessen Hilfe beispielsweise die erzeugte Farbe bzw. das erzeugte monochromatische Licht eines VCSEL in eine beliebige Farbe veränderbar ist, wodurch z. B. ein Pixel im RGB-Raum geschaffen wird.

Ferner ist bevorzugt, dass das Erstellen der wenigstens einen Funktionsschicht und/oder der wenigstens einen Schicht der Oberschicht und/oder der mindestens einen Strahlformeinrichtung ein Verschmelzen umfasst. Auf diese Weise kann ein Material durch z. B. Wärmezufuhr von einer pulverigen Form in eine fest, verschmolzene Form überführt werden. Diese Herstellung erlaubt günstigerweise das Erzeugen einer ebenen Oberfläche auf einer Schicht.

Auch ist es günstig, wenn das Erstellen der wenigstens einen Funktionsschicht und/oder der wenigstens einen Schicht der Oberschicht und/oder der mindestens einen Strahlformeinrichtung ein generatives Fertigungsverfahren, insbesondere Stereolithografie (SLA), umfasst. Hierbei kann es sich vorzugsweise auch um ein selektives Laserschmelzen (SLM) selektives Lasersintern (SLS), Selective Head Sintering (SHS), Binder Jetting, Elektronenstrahlschmelzen (EBM), Fused Deposition Modeling (FDM), Laminated Object Modelling (LOM), Cladding, Wax Deposition Modeling (WDM), Contour Crafting, Kaltgasspritzen, Stereolithografie (SLA), Digital Light Processing (DLP) und/oder Liquid Composite Moulding (LCM) handeln. Als generatives Fertigungsverfahren bzw. als additive Fertigung werden häufig Rapid Prototyping Verfahren zur schnellen und kostengünstigen Fertigung von Modellen, Mustern, Prototypen, Werkzeugen und Endprodukten bezeichnet. Bei einer derartigen Fertigung wird in der Regel direkt auf Basis von Datenmodelle aus formlosen Flüssigkeiten, Pulver usw. oder formneutralem band-, drahtförmig Material mittels chemischer und/oder physikalischer Prozesse ein Gegenstand produziert.

Es kann auch vorgesehen sein, dass das Erstellen der wenigstens einen Funktionsschicht und/oder der wenigstens einen Schicht der Oberschicht und/oder der mindestens einen Strahlformeinrichtung ein Nachbearbeiten der Oberschicht umfasst. Bei dem Nachbearbeiten der Oberschicht kann es sich beispielsweise darum handeln, eine bestimmte Oberflächenstruktur herzustellen. Insbesondere umfasst das Erstellen ein Nachbearbeiten der Oberschicht mittels eines Lasers und/oder mittels einer spanabhebenden Bearbeitung, vorzugsweise mittels Fräsen und/oder Bohren und/oder mittels eines chemischen Verfahrens, vorzugsweise mittels Ätzen, und/oder mittels Polieren, um eine Oberflächenschicht bzw. eine äußere Oberfläche zu erzeugen. Mithilfe der vorgenannten Verfahren ist es zum einen möglich, eine im Wesentlichen ebene Oberfläche bzw. eine im Wesentlichen ebene Oberflächenschicht der Oberschicht, die zu einem Betrachter gewandt ist, zu erstellen. Zum anderen kann in die Oberschicht auch ein Durchgang bzw. ein Mikro-Durchgang eingebracht werden. Dies erfolgt beispielsweise durch Fräsen und/oder Bohren und/oder Ätzen eines Durchgangs durch die Oberschicht. Auf diese Weise kann also beispielsweise ein Durchgang für Licht geschaffen werden, mithilfe dessen es zum Beispiel möglich ist, erzeugtes Licht von einer lichterzeugenden Leuchtfläche von der einen Seite der Oberschicht auf die andere zu leiten bzw. gelangen zu lassen.

Des Weiteren ist es möglich, dass das Nachbearbeiten auch ein Entfernen eines Trägers und/oder einer Trägerschicht umfasst, auf welchem bzw. welcher diverse Strukturen, Schichten und/oder Strahlformeinrichtungen auf einfache Weise aufgebaut bzw. erstellt wurden. Auch ist es denkbar, dass auf die Trägerschicht verzichtet werden kann. Dies ist günstigerweise z. B. dann der Fall, wenn die Oberschicht nicht auf der Trägerschicht bzw. einem Träger angeordnet wird bzw. ist, sondern auf dem Display der Anzeigevorrichtung oder einem Glas des Displays.

Des Weiteren ist es von Vorteil, wenn das Aufbauen der Oberschicht ein generatives Fertigungsverfahren, insbesondere ein Gießen von einem Werkstoff, umfasst. Beim Gießen wird der Werkstoff vorzugsweise nicht unter Druck in eine Form eingespritzt, sondern - ähnlich dem Gießvorgang von großen Bauteilen für z. B. Motoren - eine Gießmasse in eine Form gegossen oder auf beispielsweise eine Schicht aufgegossen. Auf diese Weise ist eine im Wesentlichen ebene oberflächenbildende Oberflächenschicht der Oberschicht bzw. eine ebene äußere Oberfläche einfach und kostengünstig erzeugbar. Ferner ist bevorzugt, wenn auch hier der gegossene Werkstoff mittels UV-Licht, zugeführter oder abgeführter Wärme ausgehärtet wird. Dies erlaubt es der Gussmasse zunächst in die Kavitäten der Form einfließen zu lassen und somit Lufteinschlüsse durch eine Zeitverzögerung nach dem Gießen des Werkstoffes zu verhindern. Auch ist es möglich, vor dem Härten mittels Licht oder Wärme die gegossene bzw. vergossene Masse Vibrationen auszusetzten, die Lufteinschlüsse nach außen befördern können. Auf diese Weise können Lufteinschlüsse - wie bereits angedeutet - verhindert und eine hochwertige Schicht erzeugt werden.

Auch ist bevorzugt, dass das Aufbauen der Oberschicht ein Füllen des Zwischenraums zwischen mindestens zwei Strahlformeinrichtungen umfasst. Diese Vorgehensweise erlaubt es beispielsweise, zunächst Strahlformeinrichtungen herzustellen, diese korrekt zueinander zu positionieren, und im Anschluss daran die einzelnen voneinander getrennten Strahlformeinrichtungen durch das Füllen der Zwischenräume zwischen den Strahlformeinrichtungen miteinander zu verbinden. Somit können also Strahlformeinrichtungen bzw. mindestens eine Strahlformeinrichtung vor dem Aufbauen der Oberschicht nicht nur hergestellt, sondern auch einer Qualitätskontrolle unterzogen werden, wobei Ausschuss beim Erstellen einer Anzeigevorrichtung vermieden werden kann.

Ferner ist es günstig, wenn die zum Füllen genutzte Menge an Material, insbesondere Kunststoff, vorteilhafterweise opaker Kunststoff, oder Metall, die mindestens eine Strahlformeinrichtung, zumindest teilweise, vorzugsweise vollständig, bedeckt. Das Bedecken der mindestens einen Strahlformeinrichtung dient dazu, in einem anschließenden Herstellungsschritt, insbesondere bei einem Nachbearbeiten der Oberschicht, Material aufzubringen, das ein Nachbearbeiten auch ermöglicht.

Es kann auch vorgesehen sein, dass die zum Füllen genutzte Menge an Material, insbesondere Kunststoff oder Metall, die mindestens eine Strahlformeinrichtung zumindest auf der zum Betrachter gewandten Oberfläche derart bedeckt, dass wenigstens ein Bereich bzw. Teilbereich des Opferelements von Material umschlossen ist. Anders ausgedrückt ist es von Vorteil, wenn das Opferelement derart von dem zum Füllen genutzten Material bedeckt wird, dass vorzugsweise bei einem Nachbearbeiten der erstellten Oberschicht bzw. der oberflächenbildenden Oberflächenschicht bzw. der zum Betrachter gewandten Oberfläche das Opferelement höchstens vollständig, vorzugsweise zu 95 %, abgetragen wird. Das Abtragen kann beispielsweise durch Fräsen und/oder Ätzen und/oder Polieren realisiert werden.

Ferner ist es günstig, wenn auf der Oberschicht eine im Wesentlichen ebene, zum Betrachter gewandte Oberfläche erstellt wird. Mit anderen Worten ausgedrückt ist es von Vorteil, wenn eine oberflächenbildende Oberflächenschicht der Oberschicht eine im Wesentlichen ebene, zum Betrachter gewandte Oberfläche aufweist. Beim Erstellen einer derartigen ebenen Oberfläche wird nicht nur ein einheitliches Äußeres der Anzeigevorrichtung erzeugt, sondern auch eine Haptik, die einem Nutzer bzw. einem Betrachter den subjektiven Eindruck verschafft, die Anzeigevorrichtung wäre aus einem einzigen Material und somit einstückig aufgebaut. Des Weiteren ist es von Vorteil eine im Wesentlichen ebene Oberfläche zu erstellen, da auf dieser weitere Schichten und/oder Oberflächenstrukturen aufgebracht werden können, die unterschiedliche Funktionen erfüllen. So ist es zum Beispiel möglich eine Oberflächenstruktur aufzubringen, die hydrophob und/oder oleophob und/oder bakteriophob und/oder lichtdurchlässig ausgebildet ist.

Vorzugsweise wird beim Erstellen der im Wesentlichen ebenen Oberfläche ein optischer Durchgang geschaffen, sodass Licht aus der mindestens einen Strahlformeinrichtung ein- und/oder austreten kann. Das Erstellen eines Durchgangs kann beispielsweise mittels Bohren und/oder Ätzen und/oder Fräsen realisiert werden. Selbstverständlich sind auch weitere abtragende Verfahren zur Herstellung eines Durchgangs denkbar.

Des Weiteren ist es günstig, wenn das Erstellen der im Wesentlichen ebenen Oberfläche ein Bearbeiten der zum Betrachter gewandten Oberfläche der Anzeigevorrichtung mittels eines abtragenden Verfahrens, insbesondere mittels Schleifens und/oder Polierens, und/oder mittels eines Lasers und/oder mittels einer spanabhebenden Bearbeitung umfasst, um vorzugsweise eine im Wesentlichen ebene Oberflächenschicht bzw. eine im Wesentlichen ebene äußere Oberfläche zu erzeugen. Anders ausgedrückt ist es günstig, wenn eine im Wesentlichen ebene oberflächenbildende Oberflächenschicht der Oberschicht bzw. eine im Wesentlichen ebene äußere Oberfläche mithilfe der genannten Verfahren erzeugt wird. Beim Erstellen einer derartigen ebenen Oberfläche bzw. Oberflächenschicht wird nicht nur ein einheitliches Äußeres der Anzeigevorrichtung erzeugt, sondern auch eine Haptik, die einem Nutzer bzw. einem Betrachter den subjektiven Eindruck verschafft, die Anzeigevorrichtung wäre aus einem einzigen Material und somit einstückig aufgebaut. Dies vermittelt einen besonders hochwertigen Verarbeitungsgrad. Auch ist es mithilfe einer derartig aufgebauten Anzeigevorrichtung möglich, diese auf Seiten der Oberflächenschicht bzw. der Oberschicht wasserdicht auszugestalten. Des Weiteren ist es von Vorteil eine im Wesentlichen ebene Oberfläche zu erstellen, da auf dieser weitere Schichten und/oder Oberflächenstrukturen aufgebracht werden können, die unterschiedliche Funktionen erfüllen. So ist es zum Beispiel möglich eine Oberflächenstruktur aufzubringen, die hydrophob und/oder oleophob und/oder bakteriophob und/oder lichtdurchlässig ausgebildet ist.

Es kann vorgesehen sein, dass das Bearbeiten der zum Betrachter gewandten Oberfläche ein Generieren einer gemeinsamen ebenen Oberfläche umfasst, wobei vorzugsweise die Oberschicht und eine Vielzahl von Strahlformeinrichtungen, insbesondere deren Ausgänge und/oder deren Opferelemente derart bearbeitet werden, dass aus den Ausgängen und/oder Opferelementen der Vielzahl von Strahlformeinrichtungen Licht austreten kann. Im Zusammenhang mit den vorgenannten Merkmalen ist es von Vorteil, wenn die zum Betrachter gewandte Oberfläche derart bearbeitet wird, dass die Ausgänge der Vielzahl von Strahlformeinrichtungen das Austreten von Licht gestatten. Auf diese Weise gelangt Licht von einer Seite der Oberschicht auf die andere bzw. vorzugsweise vom Eingang zum Ausgang und günstigerweise auch vom Ausgang zum Eingang.

Ferner ist es günstig, dass das Verhältnis der Fläche des Eingangs der mindestens einen Strahlformeinrichtung und/oder der maximalen Fläche der Strahlformeinrichtung zur Fläche des Ausgangs der mindestens einen Strahlformeinrichtung kleiner oder kleiner gleich 1:25 ist. Dieses Verhältnis erlaubt eine maximale Lichtausbeute bzw. eine maximale Lichtmenge vom Eingang zum Ausgang der mindestens einen Strahlformeinrichtung zu leiten. Somit können also Verluste auf ein Minimum reduziert werden.

Auch ist es von Vorteil, wenn das Verhältnis der Fläche der durch die Oberschicht austretenden mindestens einen Strahlformeinrichtung zur Fläche der Oberschicht kleiner oder kleiner gleich 1:10 ist, insbesondere zwischen 1:100 bis 1:94. Mithilfe dieses Verhältnisses können Anzeigevorrichtungen geschaffen werden, die eine optimale Auflösung bei einer optimalen Ausnutzung des von einer Anzeigevorrichtung zur Verfügung gestellten Raumes für Licht emittierenden Leuchtflächen bzw. für Strahlformeinrichtungen bieten.

Vorzugsweise ist die Entfernung von Eingang zu Ausgang der mindestens einen Strahlformeinrichtung im Verhältnis zu dem maximalen Durchmesser oder Diagonale am Eingang der mindestens einen Strahlformeinrichtung gleich oder kleiner 10:1, insbesondere gleich oder kleiner 1:1. Auch diese Ausgestaltung erlaubt es, Verluste zu minimieren und ein Maximum an Licht bzw. Lichtintensität bzw. Lichtmenge am Ausgang der mindestens eine Strahlformvorrichtung in Richtung eines Betrachters abzugeben.

Günstigerweise weist ein Ausgang der mindestens einen Strahlformeinrichtung einen Durchmesser oder eine Diagonale kleiner 1 mm auf, vorzugsweise kleiner 50 µm, insbesondere kleiner 30 µm, bevorzugt kleiner 20 µm, besonders bevorzugt kleiner 8 µm. Je größer die Diagonale desto einfacher ist eine Herstellung, insbesondere für großflächige Anzeigevorrichtungen, wie zum Beispiel großflächige Displays für Werbung an Flughäfen oder Bahnhöfen. Je kleiner die Diagonale desto besser ist die Auflösung für kleinflächige Anzeigevorrichtungen, wie zum Beispiel mobile Telefone und/oder Smartwatches und/oder Tablets.

Es kann auch vorgesehen sein, dass die Oberfläche und/oder der Zwischenraum zwischen mindestens zwei Strahlformeinrichtungen angepasst ist, Licht-Reflektionen zu unterbinden und/oder Licht für einen höheren Kontrast zu absorbieren und/oder den Empfang von Funksignalen für Antennen zu verbessern. Durch das Unterbinden von Licht-Reflektionen kann zum Beispiel der Kontrast erhöht werden, wodurch ein Betrachter den Eindruck eines tieferen Schwarztones erhält. Genauso verhält es sich, wenn Licht absorbiert wird. Günstigerweise kann durch die mindestens eine Strahlformeinrichtung, die zumindest an ihrer zu einem Betrachter gewandten Oberfläche ein elektrisch leitendes Material umfasst, die Oberfläche und/oder der Zwischenraum zwischen mindestens zwei Strahlformeinrichtungen angepasst werden, den Empfang von Funksignalen für Antennen zu verbessern. Denn das elektrisch leitende Material kann sowohl für den Empfang von Funksignalen aber auch für das Senden solcher genutzt werden.

Vorzugsweise ist die Oberfläche und/oder der Zwischenraum zwischen mindestens zwei Strahlformeinrichtungen angepasst, die Zusammensetzung von Luft zu messen und/oder Gas zu messen und/oder Radioaktivität zu messen. Mit anderen Worten ausgedrückt, ist es von Vorteil, wenn die Oberfläche und/oder der Zwischenraum zwischen mindestens zwei Strahlformeinrichtungen eine Sensorik aufweist, mit der Luft und/oder Gas und/oder Radioaktivität gemessen bzw. erfasst werden kann. Auf diese Weise kann also eine Anzeigevorrichtung nicht nur der Anzeige von Informationen dienen sondern auch Umweltbedingungen erfassen.

Ferner ist es günstig, wenn die Oberfläche und/oder der Zwischenraum zwischen mindestens zwei Strahlformeinrichtungen angepasst ist, Geräusche zu messen und/oder Geräusche zu emittieren und/oder Gerüche zu erfassen. Auch mithilfe dieser Ausgestaltung kann eine Anzeigevorrichtung Umweltbedingungen erfassen. Ferner können mithilfe der Emission von Geräuschen auch z. B. Warnsignale und/oder eine Freisprecheinrichtung und/oder ein Klingeln, hervorgerufen durch einen Anrufer, erzeugt werden.

Vorzugsweise ist die Oberfläche und/oder der Zwischenraum zwischen mindestens zwei Strahlformeinrichtungen angepasst, Aktoren, insbesondere Mikro-Motoren, Sende- und Empfangsantennen, bewegliche Mikro-Teile, Mikro-Schmelz-Elemente, Mikro-elektromagnetische oder magnetische Elemente, Mikro-Luftdruck oder Mikro-hydraulische Elemente, vorzugsweise ein Formgedächtnis-Material aufweisend, zu umfassen. Mithilfe der aktiv bewegbaren Elemente, wie z. B. Motoren, Formgedächtnis-Material oder Aktoren, kann eine Anzeigevorrichtung erstellt werden, deren Form durch Anlegen einer Energie, bzw. Strom/Spannung, verändert werden kann. Somit ist eine derartige Anzeigevorrichtung z. B. von einer ebenen Ausbildung in eine gekrümmte Ausbildung mit vorbestimmbarer Krümmung bringbar.

Zusammenfassend kann festgehalten werden, dass mithilfe der vorgeschriebenen Merkmale eine Anzeigevorrichtung geschaffen werden kann, deren Design, Dicke und mechanische Stabilität weit über den bisherigen Stand der Technik hinausgeht.

Dabei sorgt die komplette Anzeigevorrichtung bzw. die Anzeigevorrichtung selbst für eine erhöhte mechanische Stabilität und kann eine geringere Dicke bzw. Bautiefe gegenüber konventionellen Anzeigevorrichtungen aufweisen.

Unter anderem ist ein entscheidender Vorteil der erfindungsgemäßen Anzeigevorrichtung, dass diese konventionellen, bekannten Anzeigevorrichtungen, egal welcher Art (insbesondere LCD oder OLED oder Plasma oder FED) überlegen ist. Dies liegt in erster Linie daran, dass konventionelle Anzeigevorrichtungen eine transparente Schutzschicht, meistens aus verstärktem Glas oder Kunststoff, wie Saphir-Glas, sogenannten "Gorilla" Glas oder Panzerglas usw. mit einer Dicke von 0,5 mm bis 1,5 mm besitzen müssen. Diese transparente Schutzschicht, auf welche vorliegende Erfindung verzichten kann, trägt erheblich zum Gewicht der gesamten Anzeigevorrichtungen bei. Somit ist die erfindungsgemäße Anzeigevorrichtung in der logischen Konsequenz leichter gegenüber konventionellen.

Teil dieser Erfindung ist es ferner, dass mithilfe des hier vorgestellten Herstellungsverfahrens besondere und einmalige Design-Effekte sowie eine hohe mechanische Stabilität eine Anzeigevorrichtung erzielt werden kann. Insbesondere im ausgeschalteten Zustand oder in den Teilen dieser innovativen Anzeigevorrichtung die nicht beleuchtet sind, können sich schillernde Farbeffekte wie bei Schmetterlingen ergeben bzw. erzeugt werden. Summa summarum handelt es sich bei dem erfindungsgemäßen Herstellungsverfahren für eine Anzeigevorrichtung um ein Verfahren, das einer Anzeigevorrichtung einzigartige Design-Effekte, -Möglichkeiten und -Merkmale bietet, die bei konventionellen Anzeigevorrichtungen weder möglich noch erwünscht sind.

Ein weiteres bevorzugtes Merkmal dieser Erfindung entsteht dadurch, dass ein Schutzglas nicht mehr notwendig ist. In konventionellen Anzeigevorrichtungen sind Schutzgläser (oder Kunststoffe) relativ dick, sodass diese mechanisch eine Anzeigevorrichtung schützen. Jedoch ist ein derartiges Glas selbst wenig elastisch bzw. zerbrechlich. Deshalb sind Schutzgläser für Anzeigevorrichtungen in der Regel mindestens 0,5 mm bis 1 ,5 mm dick und teilweise sehr teuer in der Herstellung. Im Bruchfall sind diese schwer und unter hohen Kosten zu ersetzen.

Da nach dem erfindungsgemäßen Herstellungsverfahren eine Anzeigevorrichtung im Wesentlichen lichtemittierende Leuchtflächen und eine Oberschicht aufweist, besteht kein Bedarf mehr für ein dickes transparentes Schutzglas.

Dabei kann das Material zum Füllen bzw. Füllmaterial aus weniger zerbrechlichem Metall gebildet werden. Somit sind mit dem erfindungsgemäßen Herstellungsverfahren mindestens vier wichtige Merkmale einer innovativen Anzeigevorrichtung erzielbar.

Erstens, ist eine nach dem erfindungsgemäßen Verfahren hergestellte Anzeigevorrichtung weit weniger zerbrechlich als konventionellen Anzeigevorrichtungen. Wenn z.B. ein tragbares Gerät mit konventioneller Anzeigevorrichtung auf den Boden fällt, zerbricht in nahezu allen Fällen die Anzeigevorrichtung, da das Schutzglas mit dieser Anzeigevorrichtung direkt verbunden ist.

Zweitens, kann mithilfe des erfindungsgemäßen Herstellungsverfahrens eine Anzeigevorrichtung geschaffen werden, die erheblich dünner realisierbar ist im Vergleich zu konventionellen Anzeigevorrichtungen.

Eine konventionelle Anzeigevorrichtung besteht aus mehreren Schichten, die sehr dünn sein können, jedoch muss das Schutzglas (oder Kunststoff) relativ dick bleiben, um eine Schutzfunktion zu gewährleisten, bzw. um sich selbst zu schützen. Nicht so bei einer Anzeigevorrichtung nach dem erfindungsgemäßen Herstellungsverfahren. So können die lichtemittierenden Leuchtflächen extrem dünn gehalten werden (so wie bei den besten konventionellen Anzeigevorrichtungen, z.B. 100 µm bis 200 µm bei OLED), wobei das dicke Schutzglas von z.B. 700 µm mit nur 100 µm bis 300 µm Füllmaterial bzw. Metall ersetzt werden kann (inklusive Strahlformeinrichtungen).

Anhand dieses Beispiels wird klar ersichtlich, dass eine konventionelle Anzeigevorrichtung von insgesamt z.B. 1 mm, durch diese Anzeigevorrichtung nach dem erfindungsgemäßen Herstellungsverfahren mit z.B. insgesamt 250 µm ersetzt werden kann. Das stellt einen sehr großen Vorteil für tragbare Geräte, wie z.B. eine Uhr dar, bei der historisch gesehen, die Hersteller immer bestrebt waren, diese extrem dünn (und leicht) zu gestalten.

In dieser Hinsicht stellt diese Erfindung einen besonderen Vorteil für z.B. ein OLED bzw. eine OLED-Anzeigevorrichtungen dar. OLED brauchen zusätzlich ein Schutzglas um vor Feuchtigkeit und Sauerstoff geschützt zu werden.

Um diesen Schutz zu gewährleisten kann entweder das Schutzglas extrem dünn werden (weil es mit dieser Erfindung keinen mechanischen Schutz mehr erbringen muss) oder die Optik und/oder Schutzmaterial (z.B. Metall) erfüllt bzw. ersetzt direkt bei der Herstellung die schützende Funktion gegen Feuchtigkeit und Sauerstoff des Schutzglases. Das ist natürlich nicht auf OLED Anzeigevorrichtungen begrenzt, es kann genauso für Flüssigkristalle (LCD), VCSEL oder FED bzw. Plasma-Anzeigevorrichtung direkt bei der Herstellung oder danach angewendet werden.

Drittens, kann die Anzeigevorrichtung nach dem erfindungsgemäßen Verfahren erheblich leichter hergestellt werden, da Metalle wie Aluminium, Lithium oder Titan ähnlich oder sogar leichter als Glas sein können, vor allem wenn sie nur ein Bruchteil der notwendigen Dicke (bzw. Volumens) eines herkömmlichen Schutzglases aufweisen, bei gleichen oder weit höheren Festigkeit.

Viertens, kann mithilfe des erfindungsgemäßen Herstellungsverfahrens eine Anzeigevorrichtung geschaffen werden, die deutlich biegsamer ist im Vergleich zu konventionellen Anzeigevorrichtungen. Diese Biegsamkeit resultiert günstigerweise aus dem Vorteil, dass spröde bzw. zerbrechliche Materialien, wie z. B. Glas, nicht verwendet oder im Wesentlichen nicht verwendet werden. Ferner kann durch die Verwendung eines elastischen, biegsamen Materials bzw. Füllmaterials, das in den Zwischenräumen zwischen den Strahlformeinrichtungen angebracht wird/ist, ein Biegen der gesamten Anzeigevorrichtung ermöglicht werden. Auf diese Weise kann sich die Anzeigevorrichtung mithilfe des elastischen Füllmaterials in alle Richtungen verformen bzw. verbiegen. Dabei kann bei Biegung die mindestens eine Strahlformeinrichtung unverändert bleiben, wodurch deren optische Eigenschaften erhalten bleiben. Anders ausgedrückt, ist es günstig, wenn lediglich bzw. nur das Material zwischen den Strahlformeinrichtungen bzw. das Füllmaterial biegsam ist bzw. ausgebildet ist.

Somit sind also auch biegsame Displays bzw. Anzeigevorrichtung realisierbar.

Die genannten vier obigen Punkte sind insbesondere von Vorteil für alle tragbare Geräte, insbesondere in Kombination mit einer geringeren reflektiven Oberfläche (unter Sonneneinstrahlung), und größeren Energieersparnissen.

Es wird noch ausdrücklich darauf hingewiesen, dass mithilfe aller vorgenannten Merkmale nicht nur ein Verfahren zur Herstellung einer Anzeigevorrichtung, sondern ebenfalls die Anzeigevorrichtung selbst ausgebildet werden kann. Somit dienen also die vorgenannten Merkmale nicht nur als Verfahrensmerkmale, sondern auch als Vorrichtungsmerkmale, wodurch also eine Anzeigevorrichtung ausgestaltbar ist.

Anders ausgedrückt, die oben unter dem Verfahren zur Herstellung einer Anzeigevorrichtung genannten Merkmale können auch hier unter der Anzeigevorrichtung mit weiteren Merkmalen kombiniert werden.

Vorteilhafterweise umfasst die Anzeigevorrichtungen, insbesondere erhalten durch ein Verfahren mit den oben genannten Merkmalen, ein Display und eine Oberschicht. Hierbei dient günstigerweise die Oberschicht dazu, das Display zu schützen, insbesondere vor äußeren Einflüssen, die durch Stürze hervorgerufen werden.

Dabei ist günstigerweise die Oberschicht an lichtemittierenden Leuchtflächen des Displays angeordnet und weist ferner eine im Wesentlichen ebene, zu einem Betrachter gewandte Oberfläche auf. Auf diese Weise kann Licht der Leuchtflächen bestmöglich durch die Oberschicht gelangen, wobei eine im Wesentlichen ebene, zu einem Betrachter gewandte Oberfläche eine angenehme Haptik für einen Nutzer der Anzeigevorrichtungen darstellt.

Auch ist es von Vorteil, wenn die Oberschicht Mikro-Durchgänge zum Durchlassen von erzeugtem Licht der lichtemittierenden Leuchtflächen des Displays umfasst, die in der zu einem Betrachter gewandten Oberfläche Mikro-Öffnungen bilden. Somit wird es Licht, erzeugt von den Leuchtflächen, ermöglicht, durch die Oberschicht hindurchzutreten.

Des Weiteren ist es bevorzugt, dass die Oberschicht mindestens eine Strahlformeinrichtung umfasst, die einen Mikro-Durchgang bildet und einen Ein- und einen Ausgang für Licht umfasst. Erfindungsgemäß weist die die Oberschicht mindestens eine Strahlformeinrichtung auf, die ausgangsseitig ein Opferelement umfasst. Vorteilhafterweise kann das Opferelement zum Erstellen einer im Wesentlichen ebenen Oberfläche geopfert werden, ohne dass die Funktion der mindestens einen Strahlformeinrichtung in Mitleidenschaft gezogen wird.

Auch ist es günstig, wenn die mindestens eine Strahlformeinrichtung zumindest teilweise ein lichtdurchlässiges Material, insbesondere eine Glasfaser, und/oder ein elektrisch leitendes Material (8), insbesondere Kunststoff, umfasst, und vorzugsweise in die Oberschicht, die mittels eines generativen Fertigungsverfahrens aufgebaut wird, eingebettet ist.

Zudem ist es bevorzugt, dass die mindestens eine Strahlformeinrichtung ein Streuelement, insbesondere einen Diffusor, und/oder einen Kollimator und/oder einen Konzentrator, aufweist. Somit kann also mithilfe der mindestens einen Strahlformeinrichtungen Licht konzentriert, kollimiert und/oder gestreut werden.

Des Weiteren ist es günstig, wenn der Eingang an zumindest einer lichtemittierenden Leuchtfläche des Displays angeordnet ist. Vorteilhafterweise ist der Ausgang an der zu einem Betrachter gewandten Oberfläche der Oberschicht angeordnet. Somit wird die Orientierung der mindestens einen Strahlformeinrichtung bezüglich der Leuchtflächen des Displays bzw. bezüglich des Displays festgelegt.

Auch ist es möglich, dass am Ausgang der mindestens einen Strahlformeinrichtung ein Streuelement, insbesondere ein Diffusor und/oder ein Farbfilter-Element und/oder ein farbkonvertierendes Element und/oder ein Farb-Phosphor-Element, insbesondere ein Quantenpunkt, positioniert ist. Der Diffusor erlaubt eine Abgabe des Lichts aus der mindestens einen Strahlformeinrichtung in einem vorherbestimmbaren Winkelbereich. Auf diese Weise kann der Blickwinkel auf die jeweiligen Bedürfnisse für eine Anzeigevorrichtung angepasst werden. So kann beispielsweise ein enger Blickwinkel für Anzeigevorrichtungen mit sensiblen Daten geeignet sein, wohingegen ein großer Blickwinkel für z. B. eine Smartwatch von Vorteil ist. Das Farbfilter-Element, das farbkonvertierende Element und/oder das Farb-Phosphor-Element, insbesondere in Ausgestaltung als ein Quantenpunkt, können dazu dienen, Licht einer bestimmten Wellenlänge und somit einer bestimmten Farbe in Licht einer anderen Wellenlänge bzw. Farbe umzuwandeln. Dies ist vorteilhaft bei der Verwendung wenigstens eines VCSEL in der Anzeigevorrichtung. Somit kann also auch mit wenigstens einem VCSEL eine Anzeigevorrichtung geschaffen werden, die unterschiedliche Farben im RGB-Farbraum realisiert.

Auch ist es von Vorteil, wenn die Oberschicht im Wesentlichen Metall und/oder einen undurchsichtigen und/oder einen nicht-lichtreflektierenden Kunststoff bzw. Material umfasst. Vorzugsweise handelt es sich bei dem Metall und/oder dem Kunststoff einen widerstandsfähigen Werkstoff bzw. Material, das äußeren mechanischen Einflüssen, wie zum Beispiel Stürzen und/oder Kratzern und/oder Feuchtigkeit, widersteht.

Es wird noch darauf hingewiesen, dass vorzugsweise unter einen Erstellen ein Aufbauen bzw. günstigerweise unter einem Aufbauen auch ein Erstellen verstanden werden kann.

Nachstehend wird die Erfindung anhand von Ausführungsbeispielen in Verbindung mit zugehörigen Zeichnungen näher erläutert. Diese zeigen schematisch:
- Fig. 1 bis 5: ein erstes nicht erfindungsgemäßes Beispiel eines Herstellungsverfahrens einer dünnen und im Wesentlichen bruchfesten Anzeigevorrichtung in diversen Einzelschritten;
- Fig. 6a: eine Draufsicht auf einen Ausschnitt einer erstellten, nicht erfindungsgemäßen Anzeigevorrichtung;
- Fig. 6b: eine dreidimensionale Ansicht einer Strahlformeinrichtung 5 aus den Figuren 1 bis 5;
- Fig. 7 bis 11: ein erstes erfindungsgemäßes Ausführungsbeispiel eines Herstellungsverfahrens einer dünnen und im Wesentlichen bruchfesten Anzeigevorrichtung in diversen Einzelschritten; und
- Fig. 12 bis 17: ein zweites erfindungsgemäßes Ausführungsbeispiel eines Herstellungsverfahrens einer dünnen und im Wesentlichen bruchfesten Anzeigevorrichtung in diversen Einzelschritten.

In nachfolgender Beschreibung werden gleiche Bezugszeichen für gleiche Gegenstände verwendet.

Es wird darauf hingewiesen, dass die nachfolgenden Ausführungsbeispiele als mögliche Ausgestaltungsvarianten eines möglichen Herstellungsverfahrens zu verstehen sind.

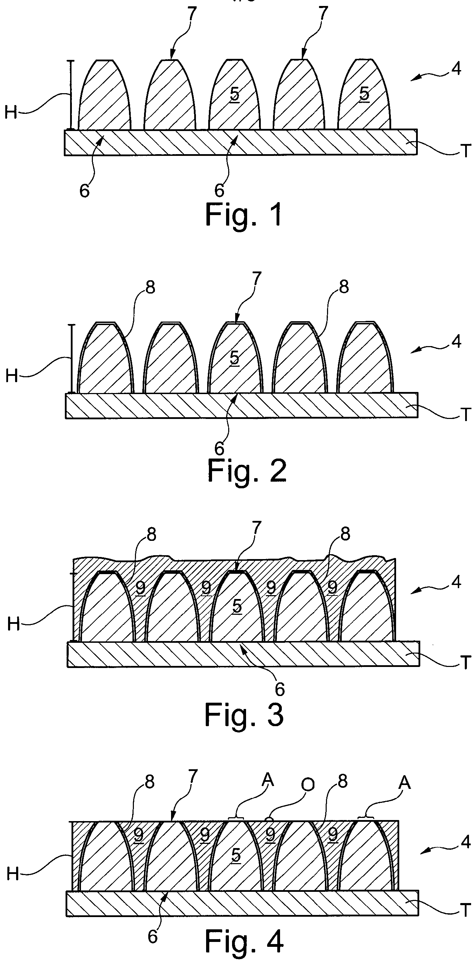

Innerhalb des in den Ansprüchen definierten Gegenstandes sind weitere Ausführungen denkbar und möglich. Figuren 1 bis 6 zeigen ein erstes nicht erfindungsgemäßes Beispiel eines Herstellungsverfahrens einer dünnen und im Wesentlichen bruchfesten Anzeigevorrichtung 1 in diversen Einzelschritten.

In genannten Figuren wird - zusammengefasst dargestellt - ein Verfahren zur Herstellung einer dünnen und im Wesentlichen bruchfesten Anzeigevorrichtung 1 mit einem Display 2 vorgestellt. Hierbei wird an lichtemittierenden Leuchtflächen 3 des Displays 2 eine Oberschicht 4 angeordnet, die eine zu einem Betrachter gewandte Oberfläche O aufweist. Die Oberschicht 4 umfasst im Wesentlichen Metall oder ein undurchsichtiges oder ein nicht-lichtreflektierendes Material.

In die Oberschicht 4 werden Mikro-Durchgänge zum Durchlassen von erzeugtem Licht der lichtemittierenden Leuchtflächen 3 des Displays 2 gebildet, die in der zu einem Betrachter gewandten Oberfläche O Mikro-Öffnungen A bilden.

Bei einem mehrere Schritte umfassenden ersten Teilabschnitt des erfindungsgemäßen Verfahrens wird die Oberschicht 4 aufgebaut.

Dies geschieht unter anderem in einem ersten Schritt - dargestellt in Figur 1 - auf einem Träger T mittels Laminated Object Modelling, einem generativen Fertigungsverfahren. Dabei werden beim Aufbauen der Oberschicht 4 - konkret dargestellt - mehrere Strahlformeinrichtungen 5 erstellt, wobei diese zumindest teilweise aus einem lichtdurchlässigen Material, insbesondere Kunststoff, erstellt werden. Diese Strahlformeinrichtungen 5 bilden innerhalb der Oberschicht 4 die Mikro-Durchgänge zum Durchlassen von Licht.

Dabei können im vorliegenden Beispiel nach Figur 1 die Strahlformeinrichtungen 5 jeweils ein Streuelement, insbesondere einen Diffusor (in Figur 1 nicht dargestellt), und/oder einen Kollimator (nicht dargestellt) und/oder einen Konzentrator aufweisen. In Figur 1 hat jede Strahlformeinrichtung 5 ferner einen parabelförmigen Querschnitt, wodurch ein Konzentrator gebildet wird. Dieser erlaubt eine Konzentration von einfallendem Licht. Dabei hat das untere Ende, das im Vergleich zum oberen Ende auf dem Träger T angeordnet ist, einen größeren Querschnitt bzw. Durchmesser.

Hierbei bildet das untere Ende jeder Strahlformeinrichtung 5, das auf dem Träger T angeordnet ist, einen Eingang 6 und das obere Ende einen Ausgang 7 für Licht.

In einem zweiten Herstellungsschritt - dargestellt in Figur 2 - wird die Oberfläche der Strahlformeinrichtungen 5 mit einem elektrisch leitenden Material 8 überzogen bzw. bedeckt bzw. beschichtet. Mit anderen Worten ausgedrückt, umfassen nun die Strahlformeinrichtungen 5 zumindest an ihrer zu einem Betrachter gewandten Oberfläche ein elektrisch leitendes Material 8. Das Material 8 kann auch zusätzlich oder nur opak ohne elektrische Leitfähigkeit ausgebildet sein.

In Figur 2 werden dabei die Strahlformeinrichtungen 5 mittels eines Dünnschicht-Verfahrens erstellt, bei dem es sich um ein Sputtern und/oder ein galvanisches Aufbringen und/oder einen Nanoimprint und/oder ein Walzenprägeverfahren und/oder ein Spritzgussverfahren handelt. Im vorliegenden Fall umfasst das Erstellen der Strahlformeinrichtungen 5 ein Sputtern mit einem Metall, insbesondere mit Aluminium. Dadurch kann zusätzlich zur elektrischen Leifähigkeit eine lichtreflektierende Fläche einfach und kostengünstig aus einem leichten Werkstoff erzeugt werden. Somit kann also Licht innerhalb des Konzentrators jeder Strahlformeinrichtung 5 bzw. innerhalb jeder Strahlformeinrichtung 5 an den von Metall bedeckten Flächen derart reflektiert werden, dass am Eingang 6 einfallendes Licht hin zum Ausgang 7 jeder Strahlformeinrichtung 5 mittels des Konzentrators konzentriert bzw. gebündelt wird.

In Figur 3 wird ein weiterer Teilschritt des Aufbauens der Oberschicht 4 schematisch gezeigt. Hierbei umfasst das Aufbauen der Oberschicht ein generatives Fertigungsverfahren, insbesondere ein Gießen von einem Werkstoff. Das Gießen bzw. Aufbauen der Oberschicht weist dabei ein Füllen der Zwischenräume 9 zwischen den Strahlformeinrichtungen 5 auf. Dadurch wird unter anderem die mechanische Stabilität erhöht.

Des Weiteren bedeckt die zum Füllen genutzte Menge an Material, welche im vorliegenden Fall vorzugsweise ein opaker Kunststoff oder Metall ist, die Strahlformeinrichtungen 5 vollständig. Wie dargestellt, kann beim Gießen keine im Wesentlichen ebene Oberfläche auf der Oberschicht 4 erstellt werden.

Daher wird in einem sich anschließenden Schritt - dargestellt in Figur 4 - auf der Oberschicht eine im Wesentlichen ebene, zum Betrachter gewandte Oberfläche O erstellt. Dabei wird beim Erstellen der im Wesentlichen ebenen Oberfläche ein optischer Durchgang geschaffen, sodass Licht aus den Strahlformeinrichtungen 5 ein- und/oder austreten kann. In der Folge werden also die Strahlformeinrichtungen 5 zumindest teilweise bzw. vollständig innerhalb der Oberschicht erstellt.

Obigen dargestellten Sachverhalt betreffend Figur 3 mit anderen Worten ausgedrückt, werden durch das Erstellen einer ebenen Oberfläche O Mikro-Öffnungen A in der Oberschicht 4 erzeugt, welche es ermöglichen, dass Licht am Ausgang 7 der Strahlformeinrichtungen 5 austreten kann.

Das Erstellen der im Wesentlichen ebenen Oberfläche O umfasst ein Bearbeiten der zum Betrachter gewandten Oberfläche O der Anzeigevorrichtung 1 mittels eines abtragenden Verfahrens, insbesondere mittels Schleifens und/oder Polierens. Auch ist es möglich, mittels eines Lasers und/oder mittels einer spanabhebenden Bearbeitung, vorzugsweise mittels Fräsen und/oder Bohren, und/oder mittels eines chemischen Verfahrens, vorzugsweise mittels Ätzens, und/oder mittels Polierens und/oder Schleifens eine Oberfläche O bzw. eine Oberflächenschicht zu erzeugen.

Zusammengefasst kann für den in Figur 4 dargestellten Schritt festgehalten werden, dass das Bearbeiten der zum Betrachter gewandten Oberfläche O ein Generieren einer gemeinsamen ebenen Oberfläche umfasst, wobei die Oberschicht 4 und eine Vielzahl von Strahlformeinrichtungen 3, insbesondere deren Ausgänge 7, derart bearbeitet werden, dass aus den Ausgängen 7 der Strahlformeinrichtungen 3 Licht austreten kann.

Generell gesagt, geschieht das Aufbauen der Oberschicht 4 mittels eines generativen Fertigungsverfahrens, wobei das Aufbauen der Oberschicht 4 neben dem Erstellen der Strahlformeinrichtungen 5 auch ein Erstellen einer weiteren Schicht und/oder wenigstens einer Funktionsschicht aufweisen kann.

Hierbei umfasst das Aufbauen der Oberschicht ein Erstellen einer oberflächenbildenden Oberflächenschicht bzw. einer äußeren Oberfläche, die die zu einem Betrachter gewandte Oberfläche O bildet. Im vorliegenden Beispiel weist die Oberschicht 4 jedoch nur eine Schicht auf, in welcher die Strahlformeinrichtungen 5 eingebettet sind. Somit entspricht also in Figur 4 die Oberschicht 4 der Oberflächenschicht.

Ferner kann das Erstellen der Oberflächenschicht, ein Aufbringen einer Oberflächenstruktur umfassen, die hydrophob und/oder oleophob und/oder bakteriophob und/oder lichtdurchlässig ausbildbar ist.

Nach dem Bearbeiten der zum Betrachter gewandten Oberfläche O durch Generieren einer gemeinsamen ebenen Oberfläche wird der Träger T ebenfalls entfernt. Dies geschieht mithilfe eines abtragenden Verfahrens, insbesondere mittels Schleifens und/oder Polierens. Auch ist es möglich, mittels eines Lasers und/oder mittels einer spanabhebenden Bearbeitung, vorzugsweise mittels Fräsen und/oder Bohren, und/oder mittels eines chemischen Verfahrens, vorzugsweise mittels Ätzens, und/oder mittels Polierens und/oder Schleifens den Träger T zu entfernen.

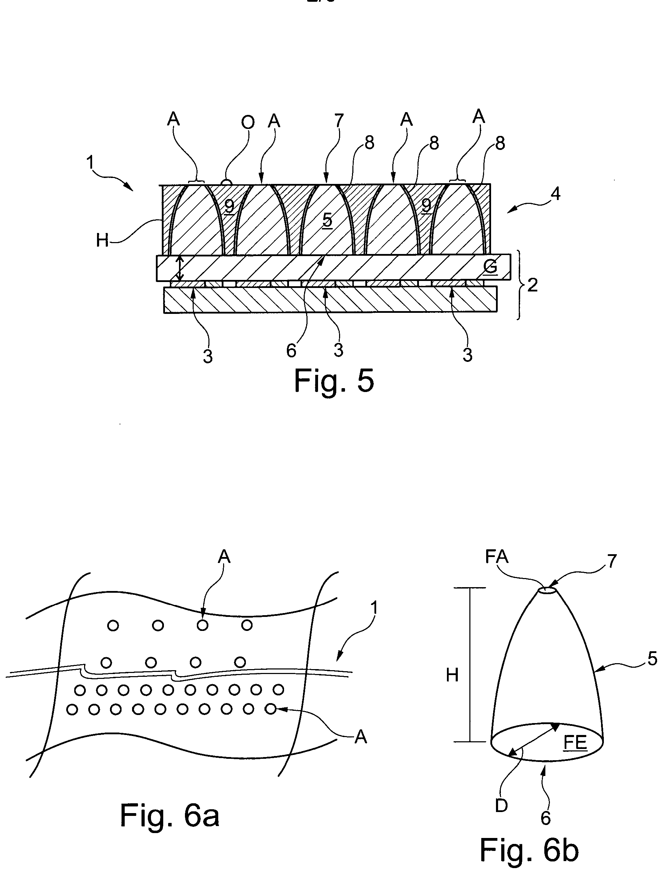

In einem weiteren Schritt - dargestellt in Figur 5 - wird das Display 2 zur Bildung der dünnen und im Wesentlichen bruchfesten Anzeigevorrichtung 1 an der Oberschicht 4 aus Figur 4 angeordnet.

Hierbei umfasst das Display 2 OLEDs oder ein Mikro-Display als lichtemittierende Leuchtflächen 3, die von einem Glasträger G geschützt sind. An diesen lichtemittierenden Leuchtflächen 3 des Displays 2 bzw. an dem Glasträger G wird die Oberschicht 4 angeordnet, die die eine zu einem Betrachter gewandte Oberfläche O aufweist.

Genauer dargestellt, sind die in der Oberschicht gebildeten Mikro-Durchgänge bzw. die die Mikro-Durchgänge bildenden Strahlformeinrichtungen 5 mit ihrem Eingang 6 an lichtemittierenden Leuchtflächen 3 des Displays 2 bzw. an dem Glasträger G angeordnet. Dabei ist idealerweise jede Strahlformeinrichtung 5 oberhalb einer lichtemittierenden Leuchtfläche 3 angeordnet, sodass ein maximales Maß an Licht der Strahlformeinrichtung zur Verfügung gestellt werden kann.

In der Folge sind die Ausgänge 7 der Strahlformeinrichtungen 5 an der zu einem Betrachter gewandten Oberfläche O der Oberschicht 4 angeordnet. Von der anderen Seite betrachtet sind die OLEDs bzw. ist das Mikro-Display 2 außerhalb der Mikro-Durchgänge, gebildet von den Strahlformeinrichtungen 5 innerhalb der Oberschicht 4, derart angeordnet, dass das von den Leuchtflächen 3 erzeugte Licht durch die Oberschicht 4 hindurchtreten kann. Anders ausgedrückt, tritt Licht von den lichtemittierenden Leuchtflächen 3 in die Eingänge 6 ein und aus den Ausgängen 7 aus. Somit dienen - wie bereits angedeutet - die Mikro-Durchgänge bzw. die die Mikro-Durchgänge bildenden Strahlformeinrichtungen 5 dem Durchlassen von Licht, sodass erzeugtes Licht der lichtemittierenden Leuchtflächen des Displays in der zu einem Betrachter gewandten Oberfläche O aus den Mikro-Öffnungen A austreten kann.

Figur 6a zeigt eine Draufsicht auf einen Ausschnitt einer erstellten, erfindungsgemäßen Anzeigevorrichtung 1.

Hierbei wurden während der Herstellung die Mikro-Öffnungen A an der zu einem Betrachter gewandten Oberfläche O der Oberschicht 4 mit einem Anteil von weniger als 10% an der gesamten zum Betrachter gewandten Oberfläche O der Oberschicht 4 erstellt bzw. eingebracht. Dabei sind im oberen Teil der Anzeigevorrichtung 1 die Mikro-Öffnungen A weiter zueinander beabstandet als im unteren Teil. Folglich sind diverse Abstände realisierbar und zugleich die Vorgabe betreffend den Anteil von weniger als 10% an der gesamten Oberfläche für die Mirko-Öffnungen gewährleistbar.

In Figur 6b ist eine dreidimensionale Ansicht einer Strahlformeinrichtung 5 aus den Figuren 1 bis 5.

Hierbei zeigt Figur 6b schematisch, dass das Verhältnis der Fläche FE des Eingangs 6 der Strahlformeinrichtung 5 bzw. das Verhältnis der maximalen Fläche der Strahlformeinrichtung 5 zur Fläche FA des Ausgangs 7 der Strahlformeinrichtung 5 kleiner 1:25 ist.

Ferner deutet Figur 6b an, dass das Verhältnis der Fläche FA der durch die Oberschicht austretenden Strahlformeinrichtung 5 zur Fläche der Oberschicht kleiner 1:100 ist.

Auch kann aus Figur 6b erkannt werden, dass die Entfernung H vom Eingang 6 zum Ausgang 7 der Strahlformeinrichtung 5 im Verhältnis zu dem maximalen Durchmesser D am Eingang 6 der Strahlformeinrichtung 5 gleich 1:1 ist.

Dabei weist der Ausgang 7 der Strahlformeinrichtung 5 einen Durchmesser kleiner 50 µm auf. Ein noch kleinerer Durchmesser ist hierbei bevorzugt.

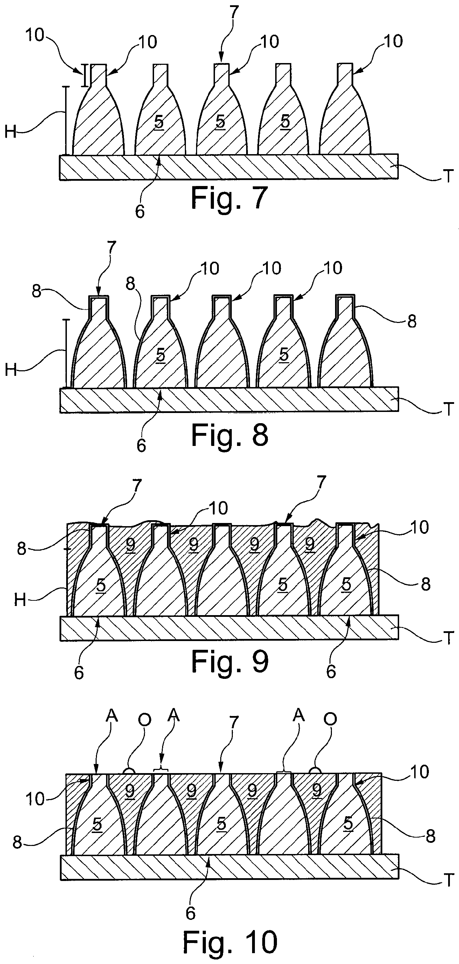

Figuren 7 bis 11 zeigen ein erstes erfindungsgemäßes Ausführungsbeispiel eines Herstellungsverfahrens einer dünnen und im Wesentlichen bruchfesten Anzeigevorrichtung in diversen Einzelschritten. Hierbei sind alle Schritte des ersten erfindungsgemäßen Ausführungsbeispiels identisch zu dem ersten nicht erfindungsgemäßen Beispiel Jedoch unterscheiden sich Strahlformeinrichtungen 5. Daher wird im nachfolgenden Text lediglich auf die Unterschiede eingegangen, ohne dass die weiteren Ausführungen, welche analog vom ersten Ausführungsbeispiel auf das zweite anwendbar sind, erneut wiederholt werden.

So ist also, detailliert geschildert, der Herstellungsschritt aus Figur 1 identisch zum Herstellungsschritt nach Figur 7, der Herstellungsschritt aus Figur 2 identisch zum Herstellungsschritt nach Figur 8, der Herstellungsschritt aus Figur 3 identisch zum Herstellungsschritt nach Figur 9, usw. Sämtliche Ausführungen, wie bereits erwähnt, zu den jeweiligen korrespondieren Schritten sind analog bzw. sogar identisch vom ersten nicht erfindungsgemäßen Beispiel auf das erste erfindungsgemäße Ausführungsbeispiel übertragbar.

Der Unterschied zwischen dem ersten nicht erfindungsgemäßen Beispiel und dem ersten erfindungsgemäßen Ausführungsbeispiel ist die Ausgestaltung der Strahlformeinrichtungen 5.

Nach dem ersten erfindungsgemäßen Ausführungsbeispiel weisen diese im Gegensatz zu den Strahlformeinrichtungen des ersten nicht erfindungsgemäßen Beispiels ausgangsseitig ein Opferelement 10 auf.

Dieses ist im vorliegenden Beispiel zylindrisch ausgebildet und erweitert eine Strahlformeinrichtung 5 nach oben, sodass in Summe die Oberschicht 4 an Dicke gewinnt. Selbstverständlich können die Opferelemente 10 der Strahlformeinrichtungen 5 weitere Formen annehmen. So können diese auch quaderförmig oder kegelförmig ausgebildet sein. Ferner wird das Opferelement 10 aus demselben Material aufgebaut wie die zuvor beschriebenen Strahlformeinrichtungen 5 und somit vorzugsweise einstückig bzw. einteilig miteinander ausgebildet. Bei dem Material handelt es sich um ein lichtdurchlässiges, welches Licht aufnehmen und weiterleiten kann.

Analog zu Figur 3 wird in Figur 9 die Oberschicht 4 mittels eines generativen Fertigungsverfahrens, insbesondere mittels Gießens eines Werkstoffs aufgebaut. Das Gießen bzw. Aufbauen der Oberschicht 4 weist dabei ein Füllen der Zwischenräume 9 zwischen den Strahlformeinrichtungen 5 auf. Dadurch wird unter anderem die mechanische Stabilität erhöht.

Hierbei bedeckt die zum Füllen genutzte Menge an Material, insbesondere Kunststoff oder Metall, die Strahlformeinrichtungen 5 zumindest auf der zum Betrachter gewandten Oberfläche O derart, dass zumindest ein Bereich des Opferelements frei von Material ist bzw. das Opferelement 10 größtenteils mit Material umgeben ist.

Bei dem sich anschließenden Schritt nach Figur 10 wird analog zu Figur 4 auf der Oberschicht 4 eine im Wesentlichen ebene, zum Betrachter gewandte Oberfläche O erstellt. Dabei wird beim Erstellen der im Wesentlichen ebenen Oberfläche O ein optischer Durchgang geschaffen, sodass Licht aus den Strahlformeinrichtungen 5 ein- und/oder austreten kann. In der Folge werden also die Strahlformeinrichtungen 5 zumindest teilweise bzw. vollständig innerhalb der Oberschicht erstellt.

Das Erstellen der im Wesentlichen ebenen Oberfläche O umfasst ein Bearbeiten der zum Betrachter gewandten Oberfläche O der Anzeigevorrichtung 1 mittels eines abtragenden Verfahrens, insbesondere mittels Schleifens und/oder Polierens. Auch ist es möglich, mittels eines Lasers und/oder mittels einer spanabhebenden Bearbeitung, vorzugsweise mittels Fräsen und/oder Bohren, und/oder mittels eines chemischen Verfahrens, vorzugsweise mittels Ätzens, und/oder mittels Polierens und/oder Schleifens eine Oberfläche O bzw. eine Oberflächenschicht zu erzeugen.

Hierbei tritt auch der Vorteil der Opferelemente 10 zu Tage. Dieser ist darin begründet, dass Fertigungstoleranzen beim Erstellen der Strahlformelemente 5 zusammen mit den Opferelementen 10 großzügiger ausgelegt werden können, wodurch Fertigungskosten gesenkt werden können.

Während beim ersten nicht erfindungsgemäßen Beispiel nach dem Gießen exakt auf die Höhe der Strahlformeinrichtungen geschliffen werden muss, um ein Maximum an Lichtausbeute zu erhalten, ist dies beim ersten erfindungsgemäßen Ausführungsbeispiel aufgrund der Opferelemente 10 nicht mehr notwendig. Denn die Höhe der Opferelemente 10 kann erfindungsgemäß für das Abtragen geopfert werden, ohne dass Einbußen beim der Lichtausbeute für Strahlformeinrichtungen 5 zu verzeichnen sind. Somit kann also beim Abtragen in Form z. B. eines Schleifvorganges eine größere Toleranz beim Abtragen verwendet werden. Dies führt ferner im Ergebnis dazu, dass ein geringer Ausschuss produziert wird und somit die Produktivität gesteigert wird.

Obige Aussagen betreffend Figur 10 anders ausgedrückt, werden beim Erstellen einer ebenen Oberfläche O Mikro-Öffnungen A in der Oberschicht 4 erzeugt, wobei diese Mikro-Öffnungen A es ermöglichen, dass Licht am von den Opferelementen 10 gebildeten Ausgang 7 der Strahlformeinrichtungen 5 austreten kann.

Zusammengefasst kann für den in Figur 10 dargestellten Schritt also konstatiert werden, dass das Bearbeiten der zum Betrachter gewandten Oberfläche O ein Generieren einer gemeinsamen ebenen Oberfläche O umfasst, wobei die Oberschicht 4 und eine Vielzahl von Strahlformeinrichtungen 3, insbesondere deren Ausgänge 7, derart bearbeitet werden, dass aus den Ausgängen 5 der Strahlformeinrichtungen 3 Licht austreten kann. Hierbei werden die Ausgänge 5 von Opferelementen 10 der Strahlformeinrichtungen 5 gebildet.

Generell kann auch hier festgehalten werden, dass das Aufbauen der Oberschicht mittels eines generativen Fertigungsverfahrens geschieht, wobei das Aufbauen der Oberschicht 4 ebenfalls ein Erstellen einer weiteren Schicht und/oder wenigstens einer Funktionsschicht und/oder das Erstellen der Strahlformeinrichtungen 5 umfasst.

Ferner wird auch hier, wie beim ersten nicht erfindungsgemäßen Beispiel nach Figur 4 nach dem Bearbeiten der zum Betrachter gewandten Oberfläche O durch Generieren einer gemeinsamen ebenen Oberfläche wird der Träger T entfernt. Dies wird mithilfe eines abtragenden Verfahrens, insbesondere mittels Schleifens und/oder Polierens vollführt. Selbstverständlich ist es auch hier möglich, mittels eines Lasers und/oder mittels einer spanabhebenden Bearbeitung, vorzugsweise mittels Fräsen und/oder Bohren, und/oder mittels eines chemischen Verfahrens, vorzugsweise mittels Ätzens, und/oder mittels Polierens und/oder Schleifens den Träger T zu entfernen.

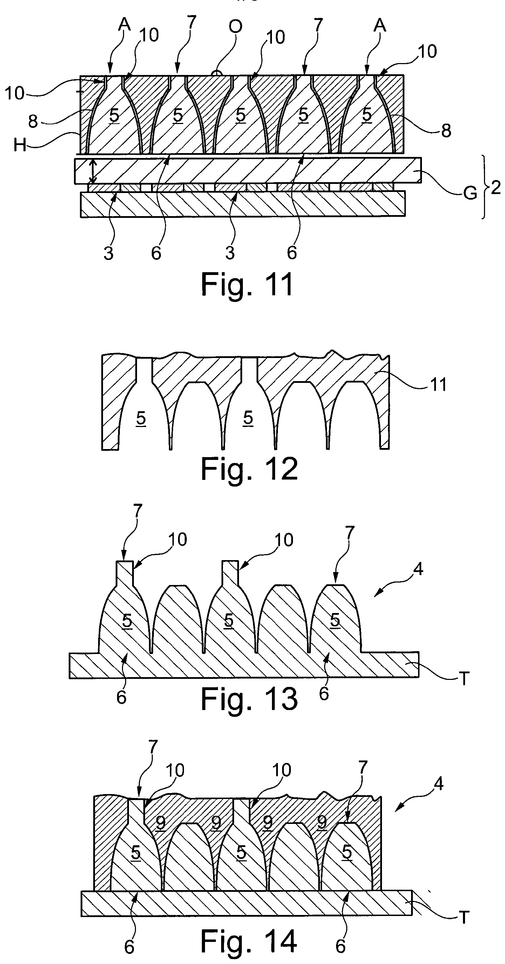

Im anschließenden Schritt - dargestellt in Figur 11 - wird das Display 2 zur Bildung der dünnen und im Wesentlichen bruchfesten Anzeigevorrichtung 1 an der Oberschicht 4 aus Figur 10 angeordnet. Es wird auf die Ausführungen nach Figur 5 verwiesen.

Figuren 12 bis 17 zeigen ein zweites erfindungsgemäßes Ausführungsbeispiel eines Herstellungsverfahrens einer dünnen und im Wesentlichen bruchfesten Anzeigevorrichtung in diversen Einzelschritten.

Hierbei wird in einem ersten Schritt, angedeutet in Figur 12, eine Matrize 11 für ein Stempelwerkzeug und/oder ein Spritzgusswerkzeug erstellt. Um die Varianten aus den vorigen beiden Ausführungsbeispielen, d.h. Strahlformeinrichtungen 5 mit und ohne Opferelement 10, gleichzeitig darzustellen, sind in Figur 12 bzw. 13 beide Varianten dargestellt. Auch sind zwei unterschiedliche Ausführungsformen für Opferelemente 10 gezeigt.

Nach dem Erstellen einer Matrize können mithilfe dieser und mittels eines Nanoimprints und/oder eines Walzenprägeverfahrens und/oder eines Spritzgussverfahrens Strahlformeinrichtungen 5, wie in Figur 12 bzw. 13 dargestellt, mit und ohne Opferelement 10 erstellt werden. Dabei sind die Strahlformeinrichtungen 5 über einen Träger T verbunden.

Unter Bezug auf Figur 13 handelt es sich bei dem ersten, linken Opferelement 10 um eine Ausführungsform, die identisch zum oben beschriebenen, ersten erfindungsgemäßen Ausführungsbeispiel ausgestaltet ist. Hingegen weist das zweite, rechte Opferelement 10 eine konische bzw. kegelförmige Form auf, die sich von unten nach oben verjüngt. Es sind also diverse Formen für Opferelemente 10 denkbar und auch realisierbar.

Wie bereits zu den beiden vorgegangen Ausführungsbeispielen erwähnt, weist eine Strahlformeinrichtung 5 auch im dritten Ausführungsbeispiel einen Ein- 6 und einen Ausgang 7 auf, wobei der Ausgang 7 nun am Opferelement 10 zu finden ist.

In Figur 14 wird mittels galvanischen Aufbringens die Oberschicht 4 weiter aufgebaut, wobei die Zwischenräume 9 zwischen den Strahlformeinrichtungen 5 aufgefüllt werden. Mithilfe dieser Maßnahme wird die mechanische Stabilität erhöht.

Ferner bedeckt nun die zum Füllen genutzte Menge an Material, welche im vorliegenden Fall ein Metall ist, vorzugsweise Titan, die Strahlformeinrichtungen 5 samt Opferelementen 10 vollständig. Wie dargestellt, kann beim galvanischen Aufbringen keine im Wesentlichen ebene Oberfläche O auf der Oberschicht 4 erstellt werden.

Idealerweise kann dem Auffüllen der Zwischenräume 9 mittels galvanischen Aufbringens ein anderer Verfahrensschritt vorausgehen. Bei diesem werden vorzugsweise die Oberflächen der Strahlformeinrichtungen 5 mit einem lichtreflektierenden Metall bedampft oder gesputtert. Dies erlaubt es den Strahlformeinrichtungen 5 die Lichtmenge von den Eingängen 6 hin zu den Ausgängen 7 zu konzentrieren, um Verluste zu reduzieren und Energie zu sparen. Das Aufbringen einer derartigen Schicht ist analog für das erste nicht erfindungsgemäße Beispiel das erste erfindungsgemäße Ausführungsbeispiel beschrieben, allerdings im Zusammenhang mit einem elektrisch leitenden Material, wobei jedoch dieses Material auch lichtreflektierend ausgebildet sein kann, wie z. B. als Aluminium.

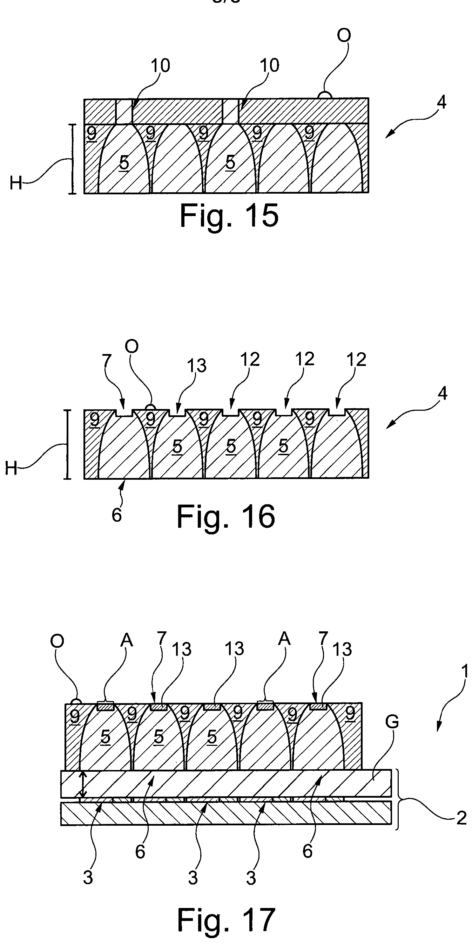

In einem sich anschließenden Schritt - dargestellt in Figur 15 - wird auf der Oberschicht 4 eine im Wesentlichen ebene, zum Betrachter gewandte Oberfläche O erstellt. Dabei wird beim Erstellen der im Wesentlichen ebenen Oberfläche ein optischer Durchgang geschaffen, sodass Licht aus den Opferelementen 10 bzw. den Ausgängen 7 der Strahlformeinrichtungen 5 austreten bzw. Licht aus den Strahlformeinrichtungen 5 ein- und/oder austreten kann. In der Folge werden also die Strahlformeinrichtungen 5 zumindest teilweise bzw. vollständig innerhalb der Oberschicht erstellt.

Des Weiteren wird der Träger T entfernt. Sowohl das Erstellen einer im Wesentlichen ebenen, zum Betrachter gewandten Oberfläche O als auch das Entfernen des Trägers T wird mittels eines Nachbearbeitens der Oberschicht 4 durchgeführt. Dabei wird das Nachbearbeiten der Oberschicht 4 mittels eines Lasers und/oder mittels einer spanabhebenden Bearbeitung, vorzugsweise mittels Fräsen und/oder Bohren, und/oder mittels eines chemischen Verfahrens, vorzugsweise mittels Ätzens, und/oder mittels Polieren, durchgeführt, um die Oberschicht 4 bzw. eine Oberflächenschicht, wenn sich die Oberschicht 4 aus mehreren Schichten zusammensetzt, zu erzeugen.

Der Einfachheit halber wird im sich anschließenden Schritt nach Figur 16 auf die Darstellung der Opferelemente 10 verzichtet. Es wird jedoch darauf hingewiesen, dass die nachfolgende Beschreibung ebenfalls auf Strahlformeinrichtungen 5 mit Opferelemente 10 zutrifft.

Gemäß Figur 16 wird im sich nach Figur 15 anschließenden Schritt die Oberschicht 4 derart nachbearbeitet, dass mittels Ätzens Ausnehmungen 11 an den Ausgängen 7 der Strahlformeinrichtungen 5 erstellt werden. Hierbei werden vor dem Ätzvorgang mittels eines auftragbaren und belichtbaren Lacks sowie mittels einer Maske und eines sich anschließenden Belichtungsvorganges Bereiche auf der Oberschicht 4 definiert, die beim Ätzen abgetragen oder geschützt werden sollen. Mit anderen Worten ausgedrückt, ist dieser Vorgang analog ausgebildet, wie bei der Mikrochipherstellung.

In die erstellten Ausnehmungen 12, die an jedem Ausgang 7 der Strahlformeinrichtungen 5 angeordnet sind, wird je ein Streuelement 13, ausgestaltet als Diffusor, positioniert. Auch ist es möglich, stattdessen oder zusätzlich einen Kollimator in einer Ausnehmung 11 zu positionieren.

Hierbei können die Streuelemente 13 durch ein Auftragen mittels eines Verspachtel-Vorganges, bei dem eine Spachtelmasse in die Mikro-Öffnungen bildenden Ausnehmungen 12 eindringt, erstellt werden.

Nachdem die Oberschicht 4 fertiggestellt wurde, kann diese in einem weiteren Schritt - dargestellt in Figur 17 - an das Display 2 zur Fertigstellung der dünnen und im Wesentlichen bruchfesten Anzeigevorrichtung 1 angeordnet werden.

Hierfür wird oberhalb der lichtemittierenden Leuchtflächen 3 des Displays 2 die Oberschicht 4 auf einem Glasträger G des Displays 2 angeordnet. Das Anordnen kann beispielsweise mit einem Klebemittel unterstützt werden, sodass die Oberschicht 4 mit dem Display 2 verbunden ist.

Genauer dargestellt, sind die in der Oberschicht gebildeten Mikro-Durchgänge bzw. die die Mikro-Durchgänge bildenden Strahlformeinrichtungen 5 mit ihrem Eingang 6 an lichtemittierenden Leuchtflächen 3 des Displays 2 bzw. oberhalb von lichtemittierenden Leuchtflächen 3 des Displays 2 angeordnet. In der Folge sind die Ausgänge 7 der Strahlformeinrichtungen 5 an der zu einem Betrachter gewandten Oberfläche O der Oberschicht 4 angeordnet.

Das Display 2 weist OLEDs oder ein Mikro-Display als lichtemittierende Leuchtflächen 3 auf. Demzufolge sind also die OLEDs bzw. das Mikro-Display außerhalb der Mikro-Durchgänge, gebildet von den Strahlformeinrichtungen 5 innerhalb der Oberschicht 4, derart angeordnet, dass das von den Leuchtflächen 3 erzeugte Licht durch die Oberschicht 4 hindurchtreten kann. Anders ausgedrückt, tritt Licht von den lichtemittierenden Leuchtflächen 3 in die Eingänge 6 einer Strahlformeinrichtung 5 ein und aus deren Ausgängen 7 aus. Somit dienen - wie bereits angedeutet - die Mikro-Durchgänge bzw. die die Mikro-Durchgänge bildenden Strahlformeinrichtungen 5 dem Durchlassen von Licht, sodass erzeugtes Licht der lichtemittierenden Leuchtflächen 3 des Displays 2 in der zu einem Betrachter gewandten Oberfläche O Mikro-Öffnungen A bildet.

Nachfolgend werden weitere Ausgestaltungen des erfindungsgemäßen Verfahrens kurz umrissen. Diese Ausführungen sind auf alle vorgestellten Ausführungsbeispiele anwendbar.

So ist es zum Beispiel möglich, dass das Aufbauen der Oberschicht 4 ein Erstellen einer weiteren Schicht und/oder wenigstens einer Funktionsschicht und/oder bzw. zusätzlich zum Erstellen von Strahlformeinrichtungen 5 umfasst. Dabei kann eine Solarschicht zur Gewinnung von Energie als wenigstens eine Funktionsschicht und/oder eine berührungsempfindliche Schicht zur Erfassung von Eingaben und/oder eine druckempfindliche Schicht zur Erfassung von Druck und/oder eine temperaturempfindliche Schicht zur Messung einer Temperatur und/oder eine kapazitive Schicht zur Messung einer Kapazität als wenigstens eine Funktionsschicht erstellt werden.

Dabei ist es denkbar, dass beim Erstellen der wenigstens einen Funktionsschicht mindestens ein sensorisches Element in die Funktionsschicht eingebracht wird, wobei vorzugsweise das mindestens eine sensorische Element als Touchsensor und/oder als Temperatursensor und/oder als Drucksensor und/oder als kapazitiver Sensor ausbildbar ist.

Auch ist es möglich, dass beim Erstellen der Strahlformeinrichtungen 5 zwischen zwei Strahlformeinrichtungen mindestens ein sensorisches Element angeordnet wird. Alternativ oder zusätzlich kann es auch sein, dass das Display bzw. dessen lichtemittierende Seite, mindestens ein sensorisches Element aufweist. Vorstellbar ist zudem, dass zwischen dem Display und der Oberschicht mindestens ein sensorisches Element aufgebracht wird.

Bei dem mindestens einen sensorischen Element kann es sich um einen Sensor handeln, insbesondere um einen zweidimensionalen und/oder dreidimensionalen Sensor, vorzugsweise einen Bildsensor und/oder einen berührungsempfindlichen und/oder einen druckempfindlichen und/oder gasempfindlichen Sensor, insbesondere ein Piezoelement.

Ferner ist es nicht zwingend, die Oberschicht 4 auf einem Träger T zu erstellen. So ist es auch möglich, die Oberschicht 4 direkt auf dem Glasträger G oder dem Display 2 bzw. dessen Leuchtfläche 3 anzuordnen. Dadurch kann eine noch dünnere und somit leichtere Anzeigevorrichtung erstellt werden.

### Bezugszeichenliste

- 1: Anzeigevorrichtung
- 2: Display
- 3: Leuchtfläche
- 4: Oberschicht
- 5: Strahlformeinrichtung
- 6: Eingang
- 7: Ausgang
- 8: elektrisch leitendes Material
- 9: Zwischenraum
- 10: Opferelement
- 11: Matratze
- 12: Ausnehmung
- 13: Streuelement

- A: Mikro-Öffnung
- T: Träger
- G: Glasträger
- FA: Fläche des Ausgangs
- FE: Fläche des Eingangs
- D: Durchmesser am Eingang
- H: Entfernung

## Patentansprüche

1. Verfahren zur Herstellung einer dünnen und im Wesentlichen bruchfesten Anzeigevorrichtung (1) mit einem Display (2),
- wobei an lichtemittierenden Leuchtflächen (3) des Displays (2) eine Oberschicht (4) angeordnet wird, die eine zu einem Betrachter gewandte Oberfläche (O) aufweist,
- wobei in der Oberschicht (4) Mikro-Durchgänge zum Durchlassen von erzeugtem Licht der lichtemittierenden Leuchtflächen (3) des Displays (2) gebildet werden, die in der zu einem Betrachter gewandten Oberfläche (O) Mikro-Öffnungen (A) bilden,
- wobei auf der Oberschicht (4) eine im Wesentlichen ebene, zum Betrachter gewandte Oberfläche (O) erstellt wird, und
- wobei die Oberschicht (4) mindestens eine Strahlformeinrichtung (5) umfasst, welche einen Mikro-Durchgang bildet,
**dadurch gekennzeichnet, das**
- die Oberschicht (4) mittels eines generativen Fertigungsverfahrens aufgebaut wird, und
- die mindestens eine Strahlformeinrichtung (5) ausgangsseitig ein Opferelement (10) zum Erstellen einer im Wesentlichen ebenen Oberfläche umfasst, welches lichtdurchlässig ist, und welches Licht aufnimmt und weiterleitet.

2. Verfahren nach Anspruch 1,
- wobei die Oberschicht (4) im Wesentlichen Metall und/oder ein undurchsichtiges und/oder nicht-lichtreflektierendes Material umfasst.

3. Verfahren nach Anspruch 1 oder 2,
**dadurch gekennzeichnet, dass**
- das Erstellen der im Wesentlichen ebenen Oberfläche (O) ein Bearbeiten der zum Betrachter gewandten Oberfläche (O) der Anzeigevorrichtung (1) mittels eines Lasers und/oder mittels einer spanabhebenden Bearbeitung umfasst, um die im Wesentlichen ebene Oberfläche (O) zu erzeugen.

4. Verfahren nach einem der Ansprüche 1 bis 3,
**dadurch gekennzeichnet, dass**
- ein Aufbauen der Oberschicht (4) ein Erstellen einer oberflächenbildenden Oberflächenschicht, die die zu einem Betrachter gewandte Oberfläche (O) bildet, umfasst,
- wobei das Erstellen der Oberflächenschicht, ein Aufbringen einer Oberflächenstruktur umfasst,
- wobei vorzugsweise die Oberflächenstruktur hydrophob und/oder oleophob und/oder bakteriophob und/oder lichtdurchlässig ausgebildet wird.

5. Verfahren nach einem der vorangehenden Ansprüche,
**dadurch gekennzeichnet, dass**
- ein Aufbauen der Oberschicht (4) ein Erstellen einer oberflächenbildenden Oberflächenschicht, die die zu einem Betrachter gewandte Oberfläche (O) bildet, umfasst,
- wobei das Erstellen der Oberflächenschicht, ein Aufbringen einer Oberflächenstruktur umfasst,
- wobei das Erstellen der Oberflächenschicht ein Einbringen eines Edelmetalls in die Oberflächenschicht zur Veredelung umfasst.

6. Verfahren nach einem der vorangehenden Ansprüche,
**dadurch gekennzeichnet, dass**
- die Mikro-Öffnungen (A) an der zu einem Betrachter gewandten Oberfläche (O) der Oberschicht (4) mit einem Anteil von weniger als 10% an der gesamten zum Betrachter gewandten Oberfläche der Oberschicht (O) eingebracht werden.

7. Verfahren nach einem der vorangehenden Ansprüche,
**dadurch gekennzeichnet, dass**
- das Display (2) wenigstens ein VCSEL umfasst,
- wobei das wenigstens eine VCSEL zumindest teilweise innerhalb eines Mikro-Durchganges eingebracht wird.

8. Verfahren nach einem der vorangehenden Ansprüche,
**dadurch gekennzeichnet, dass**
- das Display (2) wenigstens ein OLED oder wenigstens ein LED oder ein Mikro-Display umfasst,
- wobei das wenigstens eine OLED oder das wenigstens eine LED oder das Mikro-Display außerhalb eines Mikro-Durchganges angeordnet werden.

9. Verfahren nach einem der vorangehenden Ansprüche,
**dadurch gekennzeichnet, dass**
- ein Aufbauen der Oberschicht (4) ein Erstellen einer weiteren Schicht und/oder wenigstens einer Funktionsschicht umfasst.

10. Verfahren nach Anspruch 9,
**dadurch gekennzeichnet, dass**
- eine Solarschicht zur Gewinnung von Energie als wenigstens eine Funktionsschicht erstellt wird, und/oder dass
- eine berührungsempfindliche Schicht zur Erfassung von Eingaben als wenigstens eine Funktionsschicht erstellt wird, und/oder dass
- eine druckempfindliche Schicht zur Erfassung von Druck als wenigstens eine Funktionsschicht erstellt wird, und/oder dass
- eine temperaturempfindliche Schicht zur Messung einer Temperatur als wenigstens eine Funktionsschicht erstellt wird, und/oder dass
- eine kapazitive Schicht zur Messung einer Kapazität als wenigstens eine Funktionsschicht erstellt wird.

11. Verfahren nach Anspruch 9 oder 10,
**dadurch gekennzeichnet, dass**
- beim Erstellen der wenigstens einen Funktionsschicht mindestens ein sensorisches Element in die Funktionsschicht eingebracht wird,
- wobei vorzugsweise das mindestens eine sensorische Element als Touchsensor und/oder als Temperatursensor und/oder als Drucksensor und/oder als kapazitiver Sensor ausgebildet ist.

12. Verfahren nach einem der vorangehenden Ansprüche,
**dadurch gekennzeichnet, dass**
- die mindestens eine Strahlformeinrichtung (5) zumindest an ihrer zu einem Betrachter gewandten Oberfläche (O) ein elektrisch leitendes Material (8) umfasst.

13. Verfahren nach einem der vorangehenden Ansprüche,
**dadurch gekennzeichnet, dass**
- wenigstens eine Funktionsschicht und/oder wenigstens eine Schicht der Oberschicht (4) und/oder mindestens eine Strahlformeinrichtung (5) mittels eines Dünnschicht-Verfahrens erstellt wird.

14. Verfahren nach Anspruch 13,
**dadurch gekennzeichnet, dass**
- das Erstellen mittels Dünnschicht-Verfahrens ein Sputtern und/oder ein galvanisches Aufbringen und/oder einen Nanoimprint und/oder ein Walzenprägeverfahren und/oder ein Spritzgussverfahren und/oder ein Sputtern mit einem Metall, insbesondere mit Aluminium oder Gold, und/oder eine chemische oder physikalische Gasphasenabscheidung oder ein Sol-Gel-Verfahren, und/oder ein Auftragen mittels eines Verspachtel-Vorganges, bei dem eine Spachtelmasse in die Mikro-Öffnungen (A) und zumindest zum Teil in die Mikro-Durchgänge eindringt, um mindestens eine Strahlformeinrichtung (5), insbesondere einen Diffusor, zu bilden, und/oder Stereolithografie (SLA) umfasst.

15. Verfahren nach einem der vorangehenden Ansprüche,
**dadurch gekennzeichnet, dass**
- das Aufbauen der Oberschicht (4) ein Füllen des Zwischenraums (9) zwischen mindestens zwei Strahlformeinrichtungen (5) umfasst,
- wobei die zum Füllen genutzte Menge an Material, insbesondere Kunststoff oder Metall, die mindestens eine Strahlformeinrichtung (5), zumindest teilweise, vorzugsweise vollständig, bedeckt, und/oder
- wobei vorzugsweise die zum Füllen genutzte Menge an Material, insbesondere Kunststoff oder Metall, die mindestens eine Strahlformeinrichtung (5) zumindest auf der zum Betrachter gewandten Oberfläche (0) derart bedeckt, dass wenigstens ein Bereich des Opferelements (O) von Material umschlossen ist.

16. Anzeigevorrichtung erhalten durch ein Verfahren nach einem der Ansprüche 1 bis 15, aufweisend
- ein Display (2) und eine Oberschicht (4), die an lichtemittierenden Leuchtflächen (3) des Displays (2) angeordnet ist und die eine im Wesentlichen ebene, zu einem Betrachter gewandte Oberfläche (O) aufweist,
- wobei die Oberschicht (4) Mikro-Durchgänge zum Durchlassen von erzeugtem Licht der lichtemittierenden Leuchtflächen (3) des Displays (2) umfasst, die in der zu einem Betrachter gewandten Oberfläche (O) Mikro-Öffnungen (A) bilden,
- wobei die Oberschicht (4) mindestens eine Strahlformeinrichtung (5) umfasst, die einen Mikro-Durchgang bildet und einen Ein- (6) und einen Ausgang (7) für Licht sowie ausgangsseitig ein Opferelement (10) umfasst, welches während des Verfahrens teilweise abgetragen wurde zum Erstellen einer im Wesentlichen ebenen Oberfläche, ohne dass die Funktion der mindestens einen Strahlformeinrichtung in Mitleidenschaft gezogen wird.

17. Anzeigevorrichtung nach Anspruch 16,
**dadurch gekennzeichnet, dass**
- die mindestens eine Strahlformeinrichtung (5) zumindest teilweise ein lichtdurchlässiges Material, insbesondere eine Glasfaser, und/oder ein elektrisch leitendes Material (8), insbesondere Kunststoff, umfasst, und in die Oberschicht eingebettet sind.

18. Anzeigevorrichtung nach einem der Ansprüche 16 bis 17,
**dadurch gekennzeichnet, dass**
- das Verhältnis der Fläche des Eingangs (FE) der mindestens einen Strahlformeinrichtung (5) und/oder der maximalen Fläche der Strahlformeinrichtung (5) zur Fläche des Ausgangs (FA) der mindestens einen Strahlformeinrichtung (5) kleiner oder kleiner gleich 1:25 ist.

19. Anzeigevorrichtung nach einem der Ansprüche 16 bis 18,
**dadurch gekennzeichnet, dass**
- das Verhältnis der Fläche der durch die Oberschicht (4) austretenden mindestens einen Strahlformeinrichtung (5) zur Fläche der Oberschicht kleiner oder kleiner gleich 1:10 ist, insbesondere zwischen 1:100 bis 1:94.

20. Anzeigevorrichtung nach einem der Ansprüche 16 bis 19,
**dadurch gekennzeichnet, dass**
- die Entfernung (H) von Eingang (6) zu Ausgang (7) der mindestens einen Strahlformeinrichtung (5) im Verhältnis zu dem maximalen Durchmesser oder Diagonale am Eingang (D) der mindestens einen Strahlformeinrichtung (5) gleich oder kleiner 10:1 ist, insbesondere gleich oder kleiner 1:1 ist.

21. Anzeigevorrichtung nach einem der Ansprüche 16 bis 20,
**dadurch gekennzeichnet, dass**
- ein Ausgang (7) der mindestens einen Strahlformeinrichtung (5) einen Durchmesser (D) oder eine Diagonale (D) kleiner 1 mm aufweist, vorzugsweise kleiner 50 µm aufweist, insbesondere kleiner 30 µm, bevorzugt kleiner 20 µm, besonders bevorzugt kleiner 8 µm.

22. Anzeigevorrichtung nach einem der Ansprüche 16 bis 21,
**dadurch gekennzeichnet, dass**
- die mindestens eine Strahlformeinrichtung (5) angepasst ist, Lichtinformation in beide Richtungen zu leiten, insbesondere Licht vom Eingang (6) zum Ausgang (7) und vom Ausgang (7) zum Eingang (6) der mindestens einen Strahlformeinrichtung (5), und
- die Oberfläche (O) und/oder der Zwischenraum (9) zwischen mindestens zwei Strahlformeinrichtungen (5) angepasst ist, Licht-Reflektionen zu unterbinden und/oder Licht für einen höheren Kontrast zu absorbieren und/oder den Empfang von Funksignalen für Antennen zu verbessern, und/oder
- die Oberfläche (O) und/oder der Zwischenraum (9) zwischen mindestens zwei Strahlformeinrichtungen (5) angepasst ist, die Zusammensetzung von Luft zu messen und/oder Gas zu messen und/oder Radioaktivität zu messen, und/oder
- die Oberfläche (O) und/oder der Zwischenraum (9) zwischen mindestens zwei Strahlformeinrichtungen (5) angepasst ist, Geräusche zu messen und/oder Geräusche zu emittieren und/oder Gerüche zu erfassen, und/oder
- die Oberfläche (O) und/oder der Zwischenraum (9) zwischen mindestens zwei Strahlformeinrichtungen (5) angepasst ist, Aktoren, insbesondere Mikro-Motoren, Sende- und Empfangsantennen, bewegliche Mikro-Teile, Mikro-Schmelz-Elemente, Mikro-elektromagnetische oder magnetische Elemente, Mikro-Luftdruck oder Mikro-hydraulische Elemente, vorzugsweise ein Formgedächtnis-Material aufweisend, zu umfassen.

## Claims

1. A method for manufacturing a thin and substantially unbreakable display device (1) with a display (2),
- wherein an upper layer (4) is arranged on light-emitting luminous surfaces (3) of the display (2), the upper layer having a surface (O) facing a viewer,
- wherein micro-passages are formed in the upper layer (4) extending from the light emitting layer to micro-openings (A) in the display surface for allowing transmission of light generated by the light sources from the light emitting luminous surfaces (3) of the display (2) to said micro-openings in the display surface (O) facing a viewer,
- wherein a substantially planar surface (O) facing the viewer is created on the upper layer (4), and
- wherein the upper layer (4) comprises at least one beam shaping device (5), which forms a micro-passage,
**characterized in that**
- a generative production process is used to form the upper layer (4), and
- the at least one beam forming device (5) on the output side comprises a sacrificial element (10) for creating a substantially planar surface, which is translucent and which receives and transmits light.

2. The method according to claim 1,
- wherein the upper layer (4) comprises substantially metal and/or non-light reflecting material.

3. The method according to claim 1 or 2,
**characterized in that**
- the creation of the substantially planar surface (O) comprises processing the viewer facing surface (O) of the display device (1) by means of a laser and/or by machining to produce the substantially planar surface (O).

4. The method according to any of claims 1 to 3,
**characterized in that**
- a building of the upper layer (4) comprises creating a surface layer forming a surface which forms the surface (O) facing a viewer,
- wherein the creation of the surface layer comprises an application of a surface structure,
- wherein preferably the surface structure is hydrophobic and/or oleophobic and/or is bacteriophobic and/or translucent.

5. The method according to any of the preceding claims,
**characterized in that**
- a building of the upper layer (4) comprises creating a surface layer forming a surface which forms the surface (O) facing a viewer,
- wherein the creation of the surface layer comprises an application of a surface structure,
- wherein the creation of the surface layer comprises introducing a noble metal into the surface layer for finishing.

6. The method according to any of the preceding claims,
**characterized in that**
- the micro-openings (A) on the surface (O) of the upper layer (4) facing a viewer are incorporated with a proportion of less than 10% of the total surface of the upper layer (O) facing the viewer.

7. The method according to any of the preceding claims, **characterized in that**
- the display (2) comprises at least one VCSEL,
- wherein the at least one VCSEL is introduced at least partially within a micro-passage.

8. The method according to any of the preceding claims,
**characterized in that**
- the display (2) comprises at least OLED or at least one LED or a micro display,
- the at least one OLED or the at least one LED or the micro-display are arranged outside a micro-passage.

9. The method according to any of the preceding claims,
**characterized in that**
- building up the upper layer (4) comprises creating a further layer and/or at least one functional layer.

10. The method according to claim 9,
**characterized in that**
- a solar layer for generating energy as at least one functional layer is created, and/or that
- a touch-sensitive layer for detecting inputs as at least one functional layer is created, and/or that
- a pressure-sensitive layer for detecting pressure as at least one functional layer is created, and/or that
- a temperature-sensitive layer for measuring a temperature is created as at least one functional layer, and/or that
- a capacitive layer for measuring a capacity is created as at least one functional layer.

11. The method according to claim 9 or 10,
**characterized in that**
- when creating the at least one functional layer at least one sensory element is introduced into the functional layer,
- wherein preferably the at least one sensory element is designed as a touch sensor and/or as a temperature sensor and/or as a pressure sensor and/or as a capacitive sensor.

12. The method according to any of the preceding claims,
**characterized in that**
- the at least one beam forming device (5) at least on its surface (O) facing a viewer comprises an electrically conductive material (8).

13. The method according to any of the preceding claims,
**characterized in that**
- at least one functional layer and/or at least one layer of the upper layer (4) and/or at least one beam shaping device (5) is created by means of a thin-layer method.

14. The method according to claim 13,
**characterized in that**
- the production by thin-layer method comprises sputtering and/or electroplating and/or nanoimprinting and/or roll embossing and/or injection molding, and/or a sputtering with a metal, in particular with aluminum or gold, and/or a chemical or physical vapor deposition or a sol-gel process, and/or applying by means of a troweling process, in which preferably a putty penetrates into the micro-openings (A), and preferably at least partially into the micro-passages, in order to form at least one beam shaping device (5), in particular a diffuser, and/or stereolithography (SLA).

15. The method according to any of the previous claims,
**characterized in that**
- the construction of the upper layer (4) comprises filling of the intermediate space (9) between at least two beam shaping devices (5),
- wherein the quantity of material used for filling, in particular plastic or metal, which partially, preferably completely covers the at least one beam shaping device (5), and/or
- wherein preferably the quantity of material used for filling, in particular plastic or metal, covers the at least one beam shaping device (5) at least on the surface (O) facing the viewer such that at least a portion of the sacrificial element (O) is surrounded by material.

16. A display device obtained by a method according to any of claims 1 to 15, having
- a display (2) and an upper layer (4), which is arranged on light-emitting luminous surfaces (3) of the display (2), the upper layer having a surface (O) facing a viewer,
- wherein the upper layer (4) comprises micro-passages extending from the light emitting layer to micro-openings (A) in the display surface for allowing transmission of light generated by the light sources from the light emitting luminous surfaces (3) of the display (2) to said micro-openings in the display surface (O) facing a viewer,
- wherein the upper layer (4) comprises at least one beam shaping device (5), which forms a micro-passage and comprises an input (6) and an output (7) for light as well as comprising on the output side a sacrificial element (10), which during the method has been partially worn for the creation of a substantially planer surface, without the function of the at least one beam shaping device being affected.

17. The display device according to claim 16,
**characterized in that**
- the at least one beam shaping device (5) comprises at least partially a translucent material, in particular a fiber glass, and/or an electrically conductive material (8), in partiuclar plastic, and are embedded in the upper layer.

18. The display device according to any of claims 16 to 17,
**characterized in that**
- the ratio of the area of the input (FE) of the at least one beam shaping device (5) and/or the maximum area of the beam shaping device (5) to the area of of the output (FA) of the at least one beam shaping device (5) is less than or less than or equal to 1:25.

19. The display device according to any of claims 16 to 18,
**characterized in that**
- the ratio of the area of the at least one beam forming device (5) exiting the upper layer (4) to the area of the upper layer is less than or less than or equal to 1:10, in particular between 1:100 and 1:94.

20. The display device according to any of claims 16 to 19,
**characterized in that**
- the distance (H) from input (6) to output (7) of the at least one beam forming device (5) in relation to the maximum diameter or diagonal at the input (D) of the at least one beam forming device (5) is equal to or less than 10:1, in particular equal to or less than 1:1.

21. The display device according to any of claims 16 to 20,
**characterized in that**
- an output (7) of the at least one beam shaping device (5) has a diameter (D) or a diagonal (D) less than 1 mm, preferably less than 50 µm, in particular less than 30 µm, preferably less than 20 µm, especially preferably less than 8 µm.

22. The display device according to any of claims 16 to 21,
**characterized in that**
- the at least one beam shaping device (5) is adapted to guide light information in both directions, in particular light from the input (6) to the output (7) and from the output (7) to the input (6) of the at least one beam shaping device (5), and
- the surface (O) and/or the intermediate space (9) between at least two beam shaping devices (5) is adapted to prevent light reflections and/or absorb light for a higher contrast and/or to improve the reception of radio signals for antennas, and/or
- the surface (O) and/or the intermediate space (9) between at least two beam shaping devices (5) is adapted to measure the composition of air and/or gas and/or radioactivity, and/or
- the surface (O) and/or the intermediate space (9) between at least two beam shaping devices (5) is adapted to measure noises and/or to emit noise and/or detect odors, and/or
- the surface (O) and/or the intermediate space (9) between at least two beam shaping devices (5) is adapted to comprise actuators, in particular micro motors, transmitting and receiving antennas, moving micro-parts, micro-melting elements, micro-electromagnetic or magnetic elements, micro-compressed air or micro-hydraulic elements, preferably having a shape memory material.

## Revendications

1. Procédé destiné à fabriquer un dispositif d'affichage (1) mince et essentiellement résistant à la rupture avec un afficheur (2),
- dans lequel contre des surfaces luminescentes (3) émettrices de lumière de l'afficheur (2) est agencée une couche supérieure (4) qui présente une surface (O) tournée vers un observateur,
- dans lequel, dans la couche supérieure (4), des micro-passages pour laisser passer la lumière produite des surfaces luminescentes (3) émettrices de lumière de l'afficheur (2) sont formées qui forment des micro-ouvertures (A) dans la surface (O) tournée vers un observateur,
- dans lequel, sur la couche supérieure (4) est élaborée une surface (O) essentiellement plane et tournée vers l'observateur, et
- dans lequel la couche supérieure (4) comprend au moins un équipement en forme de faisceau (5), lequel forme un micro-passage,
**caractérisé en ce que**
- la couche supérieure (4) est montée au moyen d'un procédé de fabrication génératif, et
- le au moins un équipement en forme de faisceau (5) comprend du côté de la sortie un élément sacrificiel (10) pour élaborer une surface essentiellement plane, laquelle est translucide et laquelle reçoit et transmet de la lumière.

2. Procédé selon la revendication 1,
- dans lequel la couche supérieure (4) comprend essentiellement un métal et/ou un matériau opaque et/ou ne réfléchissant pas la lumière.

3. Procédé selon la revendication 1 ou 2,
**caractérisé en ce que**
- l'élaboration de la surface (O) essentiellement plane comprend un traitement de la surface (O) tournée vers un observateur du dispositif d'affichage (1) au moyen d'un laser et/ou au moyen d'un usinage par enlèvement de copeaux pour produire la surface (O) essentiellement plane.

4. Procédé selon l'une des revendications 1 à 3,
**caractérisé en ce que**
- un montage de la couche supérieure (4) comprend une élaboration d'une couche de surface formant une surface, laquelle couche de surface forme la surface (O) tournée vers un observateur,
- dans lequel l'élaboration de la couche de surface comprend une application d'une structure de surface,
- dans lequel la structure de surface est de préférence formée de façon hydrophobe et/ou oléophobe et/ou bactériophobe et/ou translucide.

5. Procédé selon l'une des revendications précédentes,
**caractérisé en ce que**
- un montage de la couche supérieure (4) comprend une élaboration d'une couche de surface formant une surface, laquelle couche de surface forme la surface (O) tournée vers un observateur,
- dans lequel l'élaboration de la couche de surface comprend une application d'une structure de surface,
- dans lequel l'élaboration de la couche de surface comprend une introduction d'un métal précieux dans la couche de surface pour un ennoblissement.

6. Procédé selon l'une des revendications précédentes,
**caractérisé en ce que**
- les micro-ouvertures (A) contre la surface (O) tournée vers un observateur d'une couche supérieure (4) sont introduites dans une proportion inférieure à 10 % contre la totalité de la surface tournée vers l'observateur de la couche supérieure (O).

7. Procédé selon l'une des revendications précédentes,
**caractérisé en ce que**
- l'afficheur (2) comprend au moins un VCSEL,
- dans lequel le au moins un VCSEL est introduit au moins partiellement à l'intérieur d'un micro-passage.

8. Procédé selon l'une des revendications précédentes,
**caractérisé en ce que**
- l'afficheur (2) comprend au moins une DELO ou au moins une DEL ou un micro-afficheur,
- dans lequel la au moins une DELO ou la au moins une DEL ou le micro-afficheur sont agencés à l'extérieur d'un micro-passage.

9. Procédé selon l'une des revendications précédentes,
**caractérisé en ce que**
- un montage de la couche supérieure (4) comprend une élaboration d'une autre couche et/ou d'au moins une couche fonctionnelle.

10. Procédé selon la revendication 9,
**caractérisé en ce que**
- une couche solaire est élaborée pour la récupération d'énergie en tant qu'au moins une couche fonctionnelle, et/ou **en ce que**
- une couche sensible au toucher est élaborée pour la détection de données en tant qu'au moins une couche fonctionnelle, et/ou **en ce que**
- une couche sensible à la pression est élaborée pour la détection d'une pression en tant qu'au moins une couche fonctionnelle, et/ou **en ce que**
- une couche sensible à la température est élaborée pour la mesure d'une température en tant qu'au moins une couche fonctionnelle, et/ou **en ce que**
- une couche capacitive est élaborée pour la mesure d'une capacité en tant qu'au moins une couche fonctionnelle.

11. Procédé selon la revendication 9 ou 10,
**caractérisé en ce que**
- lors de l'élaboration de la au moins une couche fonctionnelle, au moins un élément sensoriel est introduit dans la couche fonctionnelle,
- dans lequel le au moins un élément sensoriel est de préférence formé en tant que capteur de toucher et/ou en tant que capteur de température et/ou en tant que capteur de pression et/ou en tant que capteur capacitif.

12. Procédé selon l'une des revendications précédentes,
**caractérisé en ce que**
- le au moins un équipement en forme de faisceau (5) comprend, au moins contre sa surface (O) tournée vers un observateur, un matériau électriquement conducteur (8).

13. Procédé selon l'une des revendications précédentes,
**caractérisé en ce que**
- au moins une couche fonctionnelle et/ou au moins une couche de la couche supérieure (4) et/ou au moins un équipement en forme de faisceau (5) est élaboré(e) au moyen d'un procédé à couches minces.

14. Procédé selon la revendication 13,
**caractérisé en ce que**
- l'élaboration au moyen d'un procédé à couches minces comprend une pulvérisation cathodique et/ou un dépôt par galvanisation et/ou une nano-impression et/ou un procédé de gaufrage au rouleau et/ou un procédé de moulage par injection et/ou une pulvérisation cathodique avec un métal en particulier avec de l'aluminium ou de l'or, et/ou un dépôt en phase vapeur chimique ou physique ou un procédé sol-gel, et/ou une application au moyen d'un processus d'enduction à la spatule par lequel un mastic pénètre les micro-ouvertures (A) et au moins partiellement les micro-passages pour former au moins un équipement en forme de faisceau (5), en particulier un diffuseur, et/ou une stéréolithographie (SLA).

15. Procédé selon l'une des revendications précédentes,
**caractérisé en ce que**
- le montage de la couche supérieure (4) comprend un remplissage de la chambre intermédiaire (9) entre au moins deux équipements en forme de faisceau (5),
- dans lequel la quantité de matériau utilisée pour le remplissage, en particulier un plastique ou un métal, recouvre au moins partiellement, de préférence entièrement, le au moins un équipement en forme de faisceau (5), et/ou
- dans lequel de préférence la quantité de matériau utilisée pour le remplissage, en particulier un plastique ou un métal, recouvre le au moins un équipement en forme de faisceau (5) au moins sur la surface (O) tournée vers l'observateur, de telle sorte qu'au moins une région de l'élément sacrificiel (O) soit entourée de matériau.

16. Dispositif d'affichage obtenu par un procédé selon l'un des revendications 1 à 15, présentant
- un afficheur (2) et une couche supérieure (4) qui est agencée contre des surfaces luminescentes (3) émettrices de lumière de l'afficheur (2) et qui présente une surface (O) essentiellement plane et tournée vers un observateur,
- dans lequel la couche supérieure (4) comprend des micro-passages pour laisser passer la lumière produite des surfaces luminescentes (3) émettrices de lumière de l'afficheur (2) qui forment des micro-ouvertures (A) dans la surface (O) tournée vers un observateur,
- dans lequel la couche supérieure (4) comprend au moins un équipement en forme de faisceau (5) qui forme un micro-passage et comprend une entrée (6) et une sortie (7) pour la lumière, ainsi que du côté de la sortie un élément sacrificiel (10), lequel aura été partiellement détruit pendant le procédé pour l'élaboration d'une surface essentiellement plane, sans que la fonction du au moins un équipement en forme de faisceau n'en soit pour autant endommagée.

17. Dispositif d'affichage selon la revendication 16,
**caractérisé en ce que**
- le au moins un équipement en forme de faisceau (5) comprend au moins partiellement un matériau translucide, en particulier une fibre de verre, et/ou un matériau électriquement conducteur (8), en particulier un plastique, et sont enchâssés dans la couche supérieure.

18. Dispositif d'affichage selon l'une des revendications 16 à 17,
**caractérisé en ce que**
- le rapport de la surface de l'entrée (FE) du au moins un équipement en forme de faisceau (5) et/ou de la surface maximale de l'équipement en forme de faisceau (5) à la surface de la sortie (FA) du au moins un équipement en forme de faisceau (5) est inférieur ou inférieur égal à 1/25.

19. Dispositif d'affichage selon l'une des revendications 16 à 18,
**caractérisé en ce que**
- le rapport de la surface du au moins un équipement en forme de faisceau (5) émergeant à travers la couche supérieure (4) à la surface de la couche supérieure est inférieur ou inférieur égal à 1/10, en particulier entre 1/100 et 1/94.

20. Dispositif d'affichage selon l'une des revendications 16 à 19,
**caractérisé en ce que**
- la distance (H) de l'entrée (6) à la sortie (7) du au moins un équipement en forme de faisceau (5) rapportée au diamètre ou à la diagonale maximal(e) à l'entrée (D) du au moins un équipement en forme de faisceau (5) est inférieure ou égale à 10/1, en particulier inférieure ou égale à 1/1.

21. Dispositif d'affichage selon l'une des revendications 16 à 20,
**caractérisé en ce que**
- une sortie (7) du au moins un équipement en forme de faisceau (5) présente un diamètre (D) ou une diagonale (D) inférieur (e) à 1 mm, de préférence inférieur (e) à 50 µm, en particulier inférieur(e) à 30 µm, de façon privilégiée inférieur(e) à 20 µm, de façon particulièrement privilégiée inférieur(e) à 8 µm.

22. Dispositif d'affichage selon l'une des revendications 16 à 21,
**caractérisé en ce que**
- le au moins un équipement en forme de faisceau (5) est adapté pour guider une information de lumière dans les deux sens, en particulier la lumière de l'entrée (6) vers la sortie (7) et de la sortie (7) vers l'entrée (6) du au moins un équipement en forme de faisceau (5), et
- la surface (O) et/ou la chambre intermédiaire (9) entre au moins deux équipements en forme de faisceau (5) est adaptée pour empêcher des réflexions de la lumière et/ou pour absorber la lumière pour un contraste plus élevé et/ou pour améliorer la réception de signaux radio pour des antennes, et/ou
- la surface (O) et/ou la chambre intermédiaire (9) entre au moins deux équipements en forme de faisceau (5) est adaptée pour mesurer la composition de l'air et/ou pour mesurer un gaz et/ou pour mesurer une radioactivité, et/ou
- la surface (O) et/ou la chambre intermédiaire (9) entre au moins deux équipements en forme de faisceau (5) est adaptée pour mesurer des bruits et/ou pour émettre des bruits et/ou pour détecter des bruits, et/ou
- la surface (O) et/ou la chambre intermédiaire (9) entre au moins deux équipements en forme de faisceau (5) est adaptée pour entourer des actionneurs, en particulier des micro-moteurs, des antennes émettrices et réceptrices, des micro-parties déplaçables, des micro-éléments fusibles, des micro-éléments électromagnétiques ou magnétiques, une micro-pression de l'air ou des micro-éléments hydrauliques, présentant de préférence un matériau avec mémoire de forme.
